# EUROPEAN PATENT SPECIFICATION

(11) **EP 4 486 324 B1**
(45) Date of publication and mention of the grant of the patent: **15.04.2026**
(21) Application number: 23707747.4
(22) Date of filing: 01.03.2023
(51) Int. Cl.: A61K 31/194, A61P 25/08

(54) **NEUROPROTECTIVE COMPOSITION COMPRISING PYRUVATE AND CITRATE**
NEUROPROTEKTIVE ZUSAMMENSETZUNG EINTHALTEND PYRUVAT UND CITRAT
COMPOSITION NEUROPROTECTRICE COMPRENANT DU PYRUVATE ET DU CITRATE

(30) Priority: 02.03.2022 EP 22159659
(43) Date of publication of application: 08.01.2025
(73) Proprietor: N.V. Nutricia, 2712 HM Zoetermeer (NL)
(72) Inventor: SILVA, Jose Pablo, 3584 CT Utrecht (NL); MEEUSEN, Hester, 3584 CT Utrecht (NL)
(74) Representative: Nederlandsch Octrooibureau
(86) International application number: PCT/EP2023/055210
(87) International publication number: WO 2023/166072

(56) References cited:
- YAMAMOTO HIRO-AKI: "Fatty acids including alpha-ketoglutarate, citrate, malate, fumarate, oxaloacetate, pyruvate, and succinate inhibit neurotoxicity induced by potassium cyanide in mice", BIOSIS, BIOSCIENCES INFORMATION SERVICE, PHILADELPHIA, PA, US, 1 April 2008 (2008-04-01), XP002807042
- FASEB JOURNAL, vol. 22, April 2008 (2008-04-01), EXPERIMENTAL BIOLOGY ANNUAL MEETING; SAN DIEGO, CA, USA; APRIL 05 -09, 2008, ISSN: 0892-6638(print)
- POPOVA I ET AL: "Metabolic correction by pyruvate halts acquired epilepsy in multiple rodent models", NEUROBIOLOGY OF DISEASE, vol. 106, 2 July 2017 (2017-07-02), pages 244 - 254, XP085152910, ISSN: 0969-9961, DOI: 10.1016/J.NBD.2017.07.012
- MALKOV ANTON ET AL: "Seizure-induced reduction in glucose utilization promotes brain hypometabolism during epileptogenesis", NEUROBIOLOGY OF DISEASE, vol. 116, 1 August 2018 (2018-08-01), AMSTERDAM, NL, pages 28 - 38, XP055940447, ISSN: 0969-9961, DOI: 10.1016/j.nbd.2018.04.016
- KIM ET AL: "Pyruvate protects against kainate-induced epileptic brain damage in rats", EXPERIMENTAL NEUROLOGY, ELSEVIER, AMSTERDAM, NL, vol. 208, no. 1, 24 October 2007 (2007-10-24), pages 159 - 167, XP022313619, ISSN: 0014-4886, DOI: 10.1016/J.EXPNEUROL.2007.08.013
- HENKE CHRISTINE ET AL: "Disruption of the sodium-dependent citrate transporter SLC13A5 in mice causes alterations in brain citrate levels and neuronal network excitability in the hippocampus", NEUROBIOLOGY OF DISEASE, ELSEVIER, AMSTERDAM, NL, vol. 143, 16 July 2020 (2020-07-16), XP086233881, ISSN: 0969-9961, [retrieved on 20200716], DOI: 10.1016/J.NBD.2020.105018
- BJÖRN BJURULF ET AL: "Potassium citrate and metabolic acidosis in children with epilepsy on the ketogenic diet: a prospective controlled study", DEVELOPMENTAL MEDICINE & CHILD NEUROLOGY, HEINEMANN WILLIAM MEDICAL BOOKS, LONDON, GB, vol. 62, no. 1, 19 November 2019 (2019-11-19), pages 57 - 61, XP071198618, ISSN: 0012-1622, DOI: 10.1111/DMCN.14393

## Description

### Field of the invention

The present invention relates to compositions for use in promoting neuroprotection, particularly in patients at risk of or suffering from epileptic seizures.

### Background art

In subjects suffering from epileptic seizures there is a temporary disruption of brain function due to abnormal excessive or synchronous neuronal activity. Its manifestation may include periods of unusual behavior, sensations and sometimes loss of consciousness. Seizures cause the mitochondria to release reactive oxygen species (ROS), causing oxidative stress and cellular macromolecule dysfunction, impairing mitochondrial stability and ultimately the production of energy in the form of Adenosine Triphosphate (ATP). Overall, this increases neuronal excitability and may result in neuronal and eventually glial damage. Neuronal and glial cell death in turn triggers the abnormal growth of pro-epileptogenic neuronal networks in the brain which contributes to the progressive deterioration of epilepsy by increasing the frequency and severity of seizures and leading to behavioral and cognitive disturbances, ultimately lowering the quality of life.

Under physiological circumstances the brain is an energy-demanding organ with many processes linked to neuron and glial cell function requiring a lot of energy in the form of ATP. The brain is strongly dependent on the continuous provision of glucose. In addition to glucose, neurons are able to oxidize other fuels such as amino acids (mainly glutamate and glutamine), ketone bodies, lactate and pyruvate. During fasting for instance, ketone bodies serve as an important fuel source gradually replacing glucose during the transition from fasting to starvation.

Epilepsy, whether idiopathic (of unknown origin), hereditary (genetically transmitted) or acquired (as a consequence of other diseases, for example stroke, traumatic brain injury) is not always well-controlled yet the ketogenic diet (KD) shows good antiepileptic effects in drug-resistant epilepsy. The KD is a dietary regimen involving production of ketone bodies and the induction of nutritional ketosis. A KD is a high-fat low-carbohydrate diet in which ketone body production is promoted by extreme decrease in carbohydrate intake and intake of a lot of fat instead. The classic KD consisting of high fat, low protein and low carbohydrate, typically with weight ratios of up to 4:1 fats to the sum of proteins and carbohydrates, was first introduced in the 1920s for use in human childhood epilepsy. The use of KD was initially suggested in order to mimic the metabolic state and biochemical changes associated with fasting, since fasting was proven to possess anticonvulsant properties. The ketogenic diet is nowadays known to be useful for the treatment of intractable epilepsy and GLUT1 deficiency and the prevention of seizures of intractable epilepsy.

Use of the KD has shown a decrease in seizures in about 50% of the patients (Dutton S, Epilepsia, 49: 67-9(2008)). In drug-resistant epilepsy, which is defined as the failure of adequate trials of two antiepileptic drugs as monotherapies or in combination to achieve sustained seizure freedom, the ketogenic diet achieves more than a 50% seizure reduction in more than 50% of the patients, and 15-25% of these patients become even seizure free.

In subjects on a KD, fatty acids are used through fatty-acid oxidation in the cell's mitochondria. The brain is normally fuelled solely by glucose, lipids in the form of plasma lipoproteins as such do not cross the blood-brain barrier to a substantial degree. The liver can use long-chain fatty acids though to synthesise the three ketone bodies β-hydroxybutyrate, acetoacetate and acetone. These ketone bodies can cross the blood-brain barrier and act as a glucose substitute.

The KD is thought to result in adaptive changes to brain energy metabolism by increasing the energy production and has an effect on both neuronal function and neurotransmitter release, as well as ATP-dependent neurotransmitter clearance from the synaptic cleft and repolarisation after excitation. The increase in mitochondrial ATP production conveyed by ketones also helps the activation of inhibitory A1 adenosine receptors and further stabilization of the neurons. This is believed to help neurons to remain stable in the face of increased energy demand i.e. neuroprotective effect. Studies have suggested that ketone administration following ischaemic injury reduces the impact on the brain of the ischaemic injury (White and Venkatesh, 2011 , Critical Care 15:219). Furthermore, studies suggest that neurodegenerative diseases, such as Parkinson's disease and Alzheimer's disease, will benefit from ketone administration (Reger et al, (2004, Neurobiol Aging. 25:31, 1 -4).

Yamamoto Hiro-aki (2008, FEBS Letters) reports that seizures and subsequent brain mtDNA damage are protected by all substrates of enzymes in the Krebs cycle such as citrate or pyruvate.

Popova et al, (2017, Neurobiology of Disease 106, 244-254) reports metabolic correction by pyruvate as a potentially highly effective treatment of acquired epilepsies.

Despite the role of the KD in the prevention and/or treatment of seizures particularly in subjects with epilepsy but also in other subjects suffering from or is at risk of seizures, there remains an ongoing need to prevent and/or treat neuronal damage. Accordingly, there remains a need to develop compositions, methods and uses to improve neuroprotection in subjects suffering from or at risk of seizures.

### Summary of the invention

The inventors have observed that after administration of a neuroprotective composition comprising therapeutically effective amounts of a combination of pyruvate and citrate, or derivatives thereof, neuronal damage in a subject suffering from or at risk of seizures is prevented or reduced.

The inventors found that pyruvate increases glycolysis, respiration, and ATP production in neuroglial cells under seizure- and ketogenic diet-like conditions and unexpectedly found that this effect is potentiated in a more than additive manner by citrate. Therefore, providing pyruvate in combination with citrate synergistically helps neurons and glial cells to meet the energy demand imposed on them by epileptic seizures and provides neuroprotective effects.

The inventors found that a composition comprising therapeutically effective amounts of a combination of pyruvate and citrate is beneficial for therapeutic use in the treatment and/or prevention and/or reduction of the severity of neuronal damage by i) increasing metabolic activity, preferably in glycolysis and mitochondrial oxygen consumption rate of neurons and glial cells in the brain ii) reducing and/or preventing excitotoxity associated with or following seizures, ii) prevention and/or treatment of neuroinflammation, iii) prevention and/or delay the formation of epileptiform circuits in the brain. Worded differently, the invention pertains to the use of a neuroprotective composition comprising therapeutically effective amounts of a combination of pyruvate and citrate, or derivatives thereof, for the treatment and/or prevention of neuronal damage in a subject suffering from or at risk of seizures. The invention also relates to a method for treatment and/or prevention of neuronal damage in a subject suffering from or at risk of seizures comprising administering to the subject a neuroprotective composition comprising therapeutically effective amounts of a combination of pyruvate and citrate, or derivatives thereof. In particular, the present invention provides neuroprotective compositions, methods of use and use of said neuroprotective compositions to treat and/or prevent neuronal damage in a subject suffering from or at risk of seizures, preferably epileptic seizures. In a preferred embodiment the neuroprotective composition is for use in a subject on a ketogenic diet.

### List of Figures

The present invention will be discussed in more detail below, with reference to the attached figures.
The present invention will be discussed in more detail below, with reference to the attached figures.
Fig. 1. describes the acute effect of citrate and pyruvate on mitochondrial respiration in resting and hyperexcited (seizure-like) neuroglial cell cultures.
Fig. 2. Describes the acute effect of citrate and pyruvate on glycolysis in resting and hyperexcited (seizure-like) neuroglial cell cultures.
Fig. 3 describes the acute effect of citrate on ATP production, mitochondrial respiration, glycolysis and lactate secretion in resting and hyperexcited (seizure-like) neuroglial cell cultures.
Fig. 4 describes the chronic effect of citrate on ATP production and mitochondrial respiration of resting neuroglial cell cultures.
Fig. 5 describes the chronic effect of citrate on glycolysis and lactate secretion of resting neuroglial cell cultures.
Fig. 6 describes the acute effect of pyruvate on ATP production and mitochondrial respiration of neuroglial cell cultures under seizure- and ketogenic diet-like conditions and the role of citrate therein.
Fig. 7 describes the acute effect of pyruvate on glycolysis and lactate secretion of neuroglial cell cultures under seizure-and ketogenic diet-like conditions and the effect of citrate thereon.
Fig. 8 describes the chronic effect of pyruvate and citrate on ATP production and mitochondrial respiration of neuroglial cell cultures under seizure- and ketogenic diet-like conditions and the effect of leucine and nicotinamide riboside thereon.
Fig. 9 describes the chronic effect of pyruvate and citrate on glycolysis and lactate secretion of neuroglial cell cultures under seizure- and ketogenic diet-like conditions.
Fig. 10 describes the chronic effect of citrate on neuronal and glial cell death induced by chronic hyperexcitation.
Fig. 11 describes the chronic effect of pyruvate on neuronal and glial cell death induced by chronic hyperexcitation.
Fig. 12 describes that chronic pre-exposure of neuroglial cell cultures to low excitatory (0.1mM) Mg²⁺ creates a permanent network change characterized by heightened network excitability and activity.
Fig. 13 describes the acute effect of switching neuroglial cell cultures from high non-excitatory (0.8mM) to low excitatory (0.1mM) Mg²⁺ medium on basal glycolysis, basal mitochondrial respiration, and basal ATP production.

### List of Embodiments

1. A neuroprotective composition comprising therapeutically effective amounts of a combination of pyruvate and citrate, for use in the treatment and/or prevention of neuronal damage in a subject suffering from or at risk of seizures.
2. Neuroprotective composition for use according to clause 1 wherein the treatment and/or prevention of neuronal damage includes the prevention and/or treatment of neuroinflammation, the prevention and/or delay of the formation of epileptiform circuits in the brain.
3. Neuroprotective composition for use according to clauses 1 and 2 wherein the treatment and/or prevention of neuronal damage involves an increase in metabolic activity, preferably in glycolysis and mitochondrial oxygen consumption rate of neurons and glial cells in the brain.
4. Neuroprotective composition for use according to the preceding clauses wherein the treatment and/or prevention of neuronal damage includes a reduction of excitotoxity associated with or following seizures.
5. Neuroprotective composition for use according to the preceding clauses wherein the subject suffering from or at risk of seizures is a subject suffering from stroke, traumatic brain injury, migraine, epilepsy, Alzheimer's disease, Parkinson's disease, or conditions impairing neuronal and glial energy metabolism including Glucose transporter type 1 (Glut1) deficiency, Pyruvate dehydrogenase (PDH) deficiency, glycogen storage diseases and mitochondrial diseases.
6. Neuroprotective composition for use according to the preceding clauses, wherein the composition further comprises one or more compounds selected from leucine and nicotinamide riboside.
7. Neuroprotective composition for use according to the preceding clauses wherein the subject suffering from or at risk of seizures is adhering to a ketogenic diet, preferably a ketogenic diet with a ketogenic ratio between 1:1 and 4:1.
8. The neuroprotective composition for use according to the preceding clauses wherein the composition is a supplement to a ketogenic diet.
9. Neuroprotective composition for use according to the preceding clauses wherein the composition is a supplement to a ketogenic tube feed.
10. Neuroprotective composition for use according to clauses 7 to 9 wherein the subject suffering from or at risk of seizures is administered the neuroprotective composition and provided with between 0.1 mg and 18 mg citrate gram of citrate per kcal of ketogenic diet and between 15 mg and 25 mg gram of pyruvate per kcal of ketogenic diet.
11. Neuroprotective composition for use according to clause 10 further providing nicotinamide riboside in an amount in a range of 0.01 mg to 0.4 mg per kcal of ketogenic diet, and/or leucine in an amount of 0.02 mg to 30 mg per kcal of ketogenic diet.
12. Neuroprotective composition for use according to the preceding clauses wherein
   citrate is present in the neuroprotective composition in an amount to provide a daily dosage of 0.01 gram to 0.7 gram per kilogram bodyweight per day;
   pyruvate is present in the neuroprotective composition in an amount to provide a daily dosage of 0.15 gram to 1 gram per kilogram bodyweight per day; and

   optionally nicotinamide riboside is present in the neuroprotective composition in an amount to provide a daily dosage of 0.5 mg to 15 mg per kilogram bodyweight per day;
   and optionally leucine is present in the neuroprotective composition in an amount to provide a daily dosage of 3 mg to 30 mg per kilogram bodyweight per day.

### Detailed description of the invention

The present invention provides for neuroprotective compositions, methods and use to treat and/or prevent neuronal damage in a subject suffering from or at risk of seizures.

A neuroprotective composition comprising therapeutically effective amounts of a combination of pyruvate and citrate is found to be beneficial for use in the treatment and/or prevention of neuronal damage by i) the reduction and/or prevention of excitotoxity associated with or following seizures, ii) the prevention and/or treatment of neuroinflammation, and/or iii) the prevention and/or delay of the formation of epileptiform circuits in the brain. In some aspects the neuroprotective composition may optionally further comprise therapeutically effective amounts of nicotinamide riboside and/or leucine. In an aspect the neuroprotective composition reduces the extent of neuronal damage in the brain.

The inventors surprisingly observed that a composition comprising pyruvate and citrate increases glycolysis and glycolytic ATP production rates of neurons and glial cells under acute and chronic seizure conditions. The increase in glycolysis rates in glial cells increases lactate production thereby sustaining neuronal mitochondrial energy production in neurons under seizure conditions as well as under seizure conditions in ketogenic conditions. The inventors further observed that leucine and nicotinamide riboside both potentiate the gain in respiratory capacity of neurons and glial cells. The provision of a composition comprising pyruvate and citrate reduced the death of neurons and glial cells under seizure conditions. Neuroinflammation ensuing from the death of neurons and glial cells is prevented and/or treated therewith. Further the composition comprising pyruvate and citrate prevents and/or delays abnormal circuit integration and/or the formation of epileptiform circuits in the brain. Such abnormal circuit integration and/or the formation of epileptiform circuits is often a consequence of neuroglial cell death and ensuing neuroinflammation.

### Definitions

Throughout this application, the following terminology and abbreviations may be used.

A classical ketogenic diet comprises an amount of lipids (by weight), which may be about 4-fold the weight of the sum of proteins and digestible carbohydrates. In the context of the invention, the so-called ketogenic (weight) ratio is the weight ratio of the amount of lipid to the combined weight amounts of protein and digestible carbohydrates in the composition. The ketogenic diet referred to in the context of the invention is a dietary intervention that may be any type of ketogenic diet known in the art, preferably characterized by a ketogenic weight ratio between 1:1 and 4:1 which is the ratio of the amount of fat to the combined amounts of protein and digestible carbohydrates. Within the aforementioned (sub)ranges of ketogenic ratios of the invention, the ketogenic diet used within the context of the invention preferably comprises a lipid weight content that is at least twice the carbohydrate weight content.

The term "ketosis" as used herein preferably refers to a subject having blood ketone levels above 0.5 mmol/L. Ketone levels sustained above 0.5 mmol/L and ideally in the range of 1 to 3 mmol/L appear to offer therapeutic effects in humans [Anderson JC et al. Obes Sci Pract. 2021; 7(5):646-656)]. Hence, the subject administered with the neuroprotective composition of the invention preferably exhibits ketone levels in the aforementioned range. Levels of ketones in the blood above 10 mmol/L are associated with signs of ketoacidosis. While ketosis refers to a state of elevated ketones, ketoacidosis is a pathological and potentially life-threatening condition amongst others resulting in a decrease in blood pH and may induce a coma.

In the context of the invention a subject adhering to or taking the neuroprotective composition or a ketogenic diet is a mammal, preferably a human. Where reference is made to amounts per kilogram bodyweight this is the bodyweight of the subject using or being administered the neuroprotective composition.

The term 'seizure' as used herein refers to a period of symptoms due to abnormally excessive or synchronous neuronal activity in the brain. A seizure often occurs when the electrical activity of the brain becomes more "synchronized" as would be the case when the person is in a drowsy state. The most common and stereotypical type of seizure is convulsive (60%). A 'convulsion' is a medical condition wherein body muscles contract and relax rapidly and repeatedly, resulting in uncontrolled shaking. Epileptic seizures may include convulsions. Epilepsy is the clinical diagnosis characterized by recurrent seizures, which are the outward manifestation of excessive and/or hyper-synchronous abnormal electrical activity of neurons in the cerebral cortex of the brain.

'Excitotoxicity' as used herein refers the excessive release or abnormal accumulation of excitatory neurotransmitters such as glutamate in synapses. Glutamate-induced excitotoxicity causes the neuronal death that occurs resulting from sustained electrical excitation of neurons during seizures in epilepsy and increased glutamate levels are observed in epileptic human brain tissues. Seizures due to epilepsy, head trauma, or neurodegenerative diseases such as Alzheimer's disease, Parkinson's disease and amyotrophic lateral sclerosis (ALS) involve the excessive release of excitatory neurotransmitters and may cause neuronal death or permanent damage due to excitotoxicity.

The term "supplement", or "dietary supplement", as used herein, refers to a nutritional product that provides nutrients to an individual that may otherwise not conveniently be consumed in sufficient quantities by said individual. Supplements typically provide the selected nutrients while not representing a significant portion of the overall nutritional needs of the subject. Typically, they do not represent more than 0.1%, 1%, 5%, 10% of the daily energy need of the subject.

Derivatives as used herein include food approved complexes or salts from pyruvate and citrate. Derivatives of pyruvate and citrate according to the present invention include physiologically acceptable complexes, acids or salts thereof such as calcium, potassium, sodium, magnesium, ammonium salts and the conjugated acids pyruvic acid and citric acid. In an embodiment the derivatives of pyruvate and citrate according to the invention may include food approved esters. When the terms pyruvate and citrate are used, derivatives of said molecules are encompassed herewith.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the elements is present, unless the context clearly requires that there be one and only one of the elements. The indefinite article "a" or "an" thus usually means "at least one".

### Neuronal damage in a subject suffering from or at risk of seizures

In an aspect of the present invention, the neuroprotective composition comprising therapeutically effective amounts of a combination of pyruvate and citrate, or derivatives thereof, is for use in the treatment and/or prevention of neuronal damage in a subject suffering from or at risk of seizures, wherein the subject is suffering from a condition selected from the group of conditions including
i) neurological diseases including stroke, traumatic brain injury, migraine, epilepsy,
ii) neurodegenerative diseases including Alzheimer's disease, Parkinson's disease, age-related mild cognitive impairment, or
iii) conditions impairing neuronal and glial energy metabolism including Glucose transporter type 1 (Glut1) deficiency, Pyruvate dehydrogenase (PDH) deficiency, glycogen storage diseases and mitochondrial diseases.

In a preferred aspect, the neuroprotective composition is for use in preventing neuronal damage in a subject having intractable epilepsy. The subject may be refractory to antiepileptic drugs or ketogenic diets or a combination of both.

In a preferred embodiment the subject suffering from or at risk of seizures is on a ketogenic diet. In an aspect the ketogenic diet may be selected from the classic ketogenic diet, the MCT diet, the modified Atkins diet and the low glycemic index treatment diet. Preferably the ketogenic diet has a ketogenic ratio between 1:1 and 4:1. Said subject is a human subject in therapeutic need of ketosis.

In a further preferred embodiment the neuroprotective composition comprising therapeutically effective amounts of a combination of pyruvate and citrate, or derivatives thereof, is for use in the treatment and/or prevention of neuronal damage suffering from or at risk of seizures wherein said subject is treated with a ketogenic diet for a neurological condition selected from stroke, traumatic brain injury, migraine, epilepsy, Alzheimer's disease, Parkinson's disease, or conditions impairing neuronal and glial energy metabolism including Glucose transporter type 1 (Glut1) deficiency, Pyruvate dehydrogenase (PDH) deficiency, glycogen storage diseases and mitochondrial diseases.

### Ingredients neuroprotective composition

### Citrate

The neuroprotective composition comprises citrate, and/or derivatives thereof, in therapeutically effective amounts. Citrate (including the conjugated acid) is present in a therapeutically effective amount in the neuroprotective composition to treat and/or prevent neuronal damage. Citrate as used in the neuroprotective composition may be in the form of citric acid or a salt of potassium (K⁺), sodium (Na⁺), Magnesium (Mg²⁺) or calcium (Ca²⁺).

Citrate or citric acid is converted to isocitrate in the first step in the Krebs cycle (also known as the citric acid or TCA cycle). Citrate is found to unexpectedly increase glycolytic rates in *in vitro* neuroglial models.

In an embodiment citrate is present in a therapeutically effective dose in the neuroprotective composition to treat and/or prevent neuronal damage by increasing metabolic activity, preferably in glycolysis and mitochondrial oxygen consumption rate of neurons and glial cells in the brain. In an aspect of the invention citrate i) reduces and/or prevents excitotoxity associated with or following seizures, ii) prevents and/or treats neuroinflammation, iii) prevents and/or delays the formation of epileptiform circuits in the brain.

In a further aspect citrate provides for the treatment and/or prevention of neuronal damage by increasing in metabolic activity, preferably in glycolysis and mitochondrial oxygen consumption rate of neurons and glial cells in the brain. In an aspect of the invention citrate and pyruvate together i) reduce and/or prevent excitotoxity associated with or following seizures, ii) prevent and/or treat neuroinflammation, iii) prevent and/or delay the formation of epileptiform circuits in the brain. In an embodiment the formation of hyperexcitable epileptic neuronal networks is prevented. In an aspect of the invention the effects of citrate and pyruvate are synergistic.

Citrate is preferably present in the neuroprotective composition in an amount to provide a daily dosage of citrate in the range of 0.01 gram to 0.7 gram per kilogram bodyweight per day, preferably 0.05 gram to 0.3 gram per kilogram bodyweight per day, more preferably 0.08 gram to 0.1 gram per kilogram bodyweight per day.

In a further aspect the neuroprotective composition is used and for use together with a ketogenic diet. Citrate is preferably present in the neuroprotective composition in a range of 0.1 mg to 17 mg citrate per kcal of ketogenic diet, more preferably 0.5 mg to 12 mg citrate per kcal of ketogenic diet, even more preferably 1 mg to 8mg citrate per kcal of ketogenic diet. Citrate is preferably present in the neuroprotective composition at a dose corresponding to 0.05 wt% to 10 wt% of the total weight of the ketogenic diet, more preferably 0.3 wt% to 5 wt% of the total weight of the ketogenic diet. Any amounts of citrate equivalents or derivatives are recalculated to the equivalent weight amount of citrate, counterions excluded.

The weight ranges and percentages of citrate in the neuroprotective composition in association with a ketogenic diet are based on a daily intake of between 500 and 4000 kcal per day and a body weight between 5 kilogram and 100 kilograms.

Daily intake of citrate of less than 15 g/day for K+/Na+/Mg2+-citrate and possibly less than 20 g/day for Ca2 +-citrate is considered safe and does not give rise to a risk of electrolyte imbalances.

### Pyruvate

The neuroprotective composition comprises pyruvate in therapeutically effective amounts. Pyruvate (including the conjugated acid) is present in a therapeutically effective amount in the neuroprotective composition to treat and/or prevent neuronal damage.

The term "pyruvate" as used in here, encompasses pyruvic acid as well as pyruvate salts and precursors of pyruvate, notably precursors that can liberate pyruvic acid or a pyruvate salt by in vivo conversion, e.g. hydrolysis, of the precursor molecule. Typical examples of pyruvate precursors that can be hydrolysed to produce pyruvate or a pyruvate salt are pyruvate esters. Pyruvate can be incorporated e.g. as free acid or as its Ca, Na or K salt.

Pyruvate is known to be the end-product of the glycolysis pathway. Astrocytes convert pyruvate to lactate and provide lactate to neurons where lactate subsequently serves as substrate for mitochondrial ATP production. Pyruvate is now found to unexpectedly increase glycolytic rates in *in vitro* neuroglial models, thereby providing for an overall increase in ATP production.

In an embodiment pyruvate is present in a therapeutically effective dose in the neuroprotective composition to treat and/or prevent neuronal damage by increasing in metabolic activity, preferably in glycolysis and mitochondrial oxygen consumption rate of neurons and glial cells in the brain. In an aspect of the invention pyruvate i) reduces and/or prevents excitotoxity associated with or following seizures, ii) prevents and/or treats neuroinflammation, iii) prevents and/or delays the formation of epileptiform circuits in the brain.

In a further aspect pyruvate provides for the treatment and/or prevention of neuronal damage by increasing in metabolic activity, preferably in glycolysis and mitochondrial oxygen consumption rate of neurons and glial cells in the brain. In an aspect of the invention the combination of citric acid and pyruvate i) reduce and/or prevent excitotoxity associated with or following seizures, ii) prevent and/or treat neuroinflammation, and/or iii) prevent and/or delay the formation of epileptiform circuits in the brain. In an embodiment the formation of hyperexcitable epileptic neuronal networks is prevented. In an aspect of the invention the effects of citric acid and pyruvate are synergistic.

Pyruvate is preferably present in the neuroprotective composition in an amount to provide a daily dosage of pyruvate in the range of 0.15 gram to 1 gram per kilogram bodyweight per day, preferably 0.3 gram to 0.6 gram per kilogram bodyweight per day, more preferably 0.4 gram to 0.5 gram per kilogram bodyweight per day.

In a further aspect the neuroprotective composition may be used and is for use together with a ketogenic diet. Pyruvate is preferably present in the neuroprotective composition in a range of 1.5 mg to 25 mg pyruvate per kcal of ketogenic diet, more preferably 2.5 mg to 15 mg pyruvate per kcal of ketogenic diet, even more preferably 5.8 mg to 12 mg pyruvate per kcal of ketogenic diet.

Pyruvate is preferably present in the neuroprotective composition at a dose corresponding to 1 wt% to 13 wt% of the total weight of the ketogenic diet, more preferably 1.5 wt% to 9 wt% of the total weight of the ketogenic diet. Any amounts of pyruvate equivalents or derivatives are recalculated to the equivalent weight amount of pyruvate, counter-ions excluded.

The weight ranges and percentages of pyruvate in the neuroprotective composition in association with a ketogenic diet are based on a daily intake of between 500 and 4000 kcal per day and a body weight between 5 kilogram and 100 kilograms.

### Nicotinamide riboside

The present invention provides neuroprotective compositions optionally further comprising a NAD+ precursor in therapeutically effective amounts, the NAD+ precursor being selected from the group consisting of tryptophan, nicotinic acid (niacin), nicotinamide (niacinamide or NAM), nicotinic acid riboside (NaR), nicotinamide riboside (NR), and mixtures thereof. In a preferred aspect the neuroprotective compositions may comprise nicotinamide riboside (NR). Any amounts of NAD+ precursors selected from the group of tryptophan, nicotinic acid (niacin), nicotinamide (niacinamide or NAM), nicotinic acid riboside (NaR), nicotinamide riboside (NR), and mixtures thereof are recalculated to the equivalent weight amount of nicotinamide riboside (NR).

As used herein, "nicotinamide riboside" includes derivatives thereof such as L-valine and L-phenylalanine esters of nicotinamide riboside.

In an embodiment there is thus provided a neuroprotective composition comprising therapeutically effective amounts of a combination of pyruvate and citrate, for use in the treatment and/or prevention of neuronal damage in a subject suffering from or at risk of seizures, wherein the neuroprotective composition further comprises nicotinamide riboside in therapeutically effective amounts.

In an embodiment, when nicotinamide riboside is present in the neuroprotective composition, the nicotinamide riboside is present in therapeutically effective amount to treat and/or prevent neuronal damage in a subject suffering from or at risk of seizures.

Nicotinamide riboside is preferably present in the neuroprotective composition in an amount to provide a daily dosage of nicotinamide riboside in the range of 0.5 mg to 15 mg per kilogram bodyweight per day, preferably 1.5 mg to 4.5 mg per kilogram bodyweight per day, more preferably 2.5 to 3.5 mg per kilogram bodyweight per day.

In a further aspect the neuroprotective composition may be used and is for use together with a ketogenic diet. Nicotinamide riboside is, when present, preferably present in the neuroprotective composition in a range of 0.01 mg to 0.4 mg nicotinamide riboside per kcal of ketogenic diet, more preferably 0.015 mg to 0.25 mg nicotinamide riboside per kcal of ketogenic diet, even more preferably 0.02 mg to 0.1 mg nicotinamide riboside per kcal of ketogenic diet. Nicotinamide riboside is preferably present in the neuroprotective composition at a dose corresponding to 0.005 wt% to 0.25 wt% of the total weight of the ketogenic diet, more preferably 0.01 wt% to 0.1 wt% of the total weight of the ketogenic diet.

### Leucine

The present invention provides neuroprotective compositions optionally further comprising leucine in therapeutically effective amounts. In the context of the invention, the terms 'leucine' and 'L-leucine' are used interchangeably. In a particular embodiment, the neuroprotective composition comprises leucine in the form of a free acid or a salt.

In an embodiment, when leucine is present in the neuroprotective composition, leucine is present in therapeutically effective amounts to treat and/or prevent neuronal damage in a subject suffering from or at risk of seizures.

In an embodiment there is thus provided a neuroprotective composition comprising therapeutically effective amounts of a combination of pyruvate and citrate, for use in the treatment and/or prevention of neuronal damage in a subject suffering from or at risk of seizures, wherein the neuroprotective composition optionally further comprises nicotinamide riboside and/or leucine.

Leucine is preferably present in the neuroprotective composition in an amount to provide a daily dosage in the range of 3 mg to 30 mg per kilogram bodyweight per day, preferably 10 mg to 25 mg per kilogram bodyweight per day, more preferably 15 mg to 20 mg per kilogram bodyweight per day. The above amounts apply to all leucine provided by the composition, including free leucine.

In a further aspect the neuroprotective composition may be used and is for use together with a ketogenic diet. Leucine is preferably present in the neuroprotective composition in a range of 0.2 mg to 30 mg leucine per kcal of ketogenic diet, more preferably 1 mg to 20 mg leucine per kcal of ketogenic diet, even more preferably 0.15 mg to 10 mg leucine per kcal of ketogenic diet. Leucine is preferably present in the neuroprotective composition at a dose corresponding to 0.02 wt% to 3 wt% of the total weight of the ketogenic diet, more preferably 0.1 wt% to 2 wt% of the total weight of the ketogenic diet.

### Neuroprotective composition

In an embodiment there is thus provided a neuroprotective composition comprising therapeutically effective amounts of a combination of pyruvate and citrate, for use in the treatment and/or prevention of neuronal damage in a subject suffering from or at risk of seizures, wherein
citrate is present in an amount to provide a daily dosage of 0.01 gram to 0.7 gram per kilogram bodyweight per day;
pyruvate is present in an amount to provide a daily dosage of 0.15 gram to 1 gram per kilogram bodyweight per day;
optionally nicotinamide riboside is present in an amount to provide a daily dosage of 0.5 mg to 15 mg per kilogram bodyweight per day;
and wherein optionally leucine is present in an amount to provide a daily dosage of 3 mg to 30 mg per kilogram bodyweight per day.

In a further embodiment there is provided a neuroprotective composition comprising therapeutically effective amounts of a combination of pyruvate and citrate, for use in the treatment and/or prevention of neuronal damage in a subject suffering from or at risk of seizures, wherein the subject suffering from or at risk of seizures is adhering to a ketogenic diet and wherein in the neuroprotective composition
citrate is provided in an amount in a range of 0.1 mg to 17 mg citrate per kcal of ketogenic diet, pyruvate is provided in an amount in a range of 1.5 mg to 25 mg pyruvate per kcal of ketogenic diet, optionally nicotinamide riboside is provided in an amount in a range of 0.01 mg to 0.4 mg per kcal of ketogenic diet,
and optionally leucine is present in a range of 0.02 mg to 30 mg per kcal of ketogenic diet.

In preferred aspects nicotinamide and/or leucine are not optional and present in the neuroprotective composition.

The weight ranges and percentages of the neuroprotective composition in association with a ketogenic diet are based on a daily intake of between 500 and 4000 kcal per day and a body weight between 5 kilogram and 100 kilograms.

In one aspect of the invention there may be provided a kit of parts comprising the neuroprotective composition and a ketogenic diet. In a preferred aspect there is provided a kit of parts comprising the neuroprotective composition and a ketogenic diet for use in the treatment and/or prevention of neuronal damage in a subject suffering from or at risk of seizures, wherein the subject suffering from or at risk of seizures is adhering to a ketogenic diet. There is thus provided for a kit of parts comprising 1) the neuroprotective composition and 2) one or more nutritional products having a ketogenic ratio between 1:1 and 4:1.

### Neuroprotective composition administration forms

The neuroprotective composition of the invention may be in any form suitable for enteral, oral or parenteral administration. Non-limiting examples of parenteral administration include intravenously, intramuscularly, intraperitoneally, subcutaneously, intraarticularly, intrasynovially, intraocularly, intrathecally, topically, and inhalation. In a preferred embodiment the neuroprotective composition is in a form suitable for oral administration.

In one aspect of the present invention, the composition according to the invention may be used as a pharmaceutical product comprising one or more pharmaceutically acceptable carrier materials.

The neuroprotective composition according to the invention may be used as a nutritional product, for example as a nutritional supplement, e.g., as an additive to the ketogenic diet, as a fortifier, to add to the ketogenic diet.

The supplement, preferably for enteral application, may be a solid or liquid galenical formulation. Examples of solid galenical formulations are tablets, capsules (e.g. hard or soft shell gelatine capsules), pills, sachets, powders, granules and the like which contain the active ingredient together with conventional galenical carriers. Any conventional carrier material can be utilized. The carrier material can be an organic or inorganic inert carrier material suitable for oral administration. Suitable carriers include water, gelatine, gum Arabic, lactose, starch, magnesium stearate, talc, vegetable oils, and the like. Additionally, additives such as flavouring agents, preservatives, stabilizers, emulsifying agents, buffers and the like may be added in accordance with accepted practices of nutritional and pharmaceutical compounding.

The neuroprotective composition according to the invention may also be used as a nutritional product for use together or co-administered with tube feeding, e.g. as an additive to a tube feed for intermittent or continuous administration.

The neuroprotective composition may contain the daily dosage in one or more dosage units. The dosage unit may be in a liquid form or in a solid form, wherein in the latter case the daily dosage may be provided by one or more solid dosage units, e.g. in one or more capsules or tablets. The neuroprotective composition can be administered at the same time as the ketogenic diet or separated by a time interval or continuous .

In an embodiment, dosing of the neuroprotective composition is at least daily; for example, a subject may receive one or more doses daily, preferably 1 to 6 times per day, more preferably 3 to 4 times per day. In some embodiments, the administration continues for the remaining life of the individual. The ideal duration of the administration of the composition can be determined by those of skill in the art.

In one aspect the neuroprotective composition combines particularly well with a 80:20 C10:C8 triglyceride composition.

Further there is provided for a kit of parts comprising the neuroprotective composition and a ketogenic diet.

The present invention has been described above with reference to a number of exemplary embodiments as shown in the drawings. Modifications and alternative implementations of some parts or elements are possible and are included in the scope of protection as defined in the appended claims.

### Examples

For a more complete understanding of the present disclosure, reference is now made to the following examples taken in conjunction with the accompanying drawings.

### Example 1

In the below described Seahorse assay it was found that pyruvate increases glycolysis, respiration, and ATP production in neuroglial cells under seizure- and ketogenic diet-like conditions and that this effect is potentiated in a more than additive manner by citrate. Therefore, providing pyruvate in combination with citrate synergistically helps neurons and glial cells to meet the energy demand imposed on them by epileptic seizures and provides neuroprotective effects.

A Seahorse assay was performed on primary rat neuronal-glial cell cultures to determine the impact of pyruvate and citric acid on glycolytic rates and lactate delivery, mitochondrial respiration and total (glycolytic and mitochondrial) ATP production.

### Primary neuroglial cell culture

Rat cortical cells were isolated from embryonic day 18 embryos using a combination of enzymatic and mechanical dissociation of brain cortical rat tissue. Immediately after dissociation, the cells were seeded at a density of 500'000 cells per mL in 96-well plates and grown at 37 °C for two weeks from day *in vitro* (DIV) 0 until DIV14 in Neurobasal medium supplemented with 2% (v/v) B27, 0.8mM Magnesium (Mg2+), 25mM glucose and 1% (v/v) Penicillin/Streptomycin. During this time neuronal axons grew out and formed synaptic contacts. In addition, glial cells, foremost astrocytes, grew and formed contacts with neurons. The incubation in 25mM glucose accelerated the differentiation and network growth of neurons.

From DIV14 until DIV17, the cells were incubated in Neurobasal medium supplemented with 2% (v/v) B27, 0.8mM Mg2+, 5mM glucose and 1% (v/v) Penicillin/Streptomycin. The switch from high to low glucose was done to approach the low intracerebral glucose concentration of the human brain and create a more physiological metabolic environment.

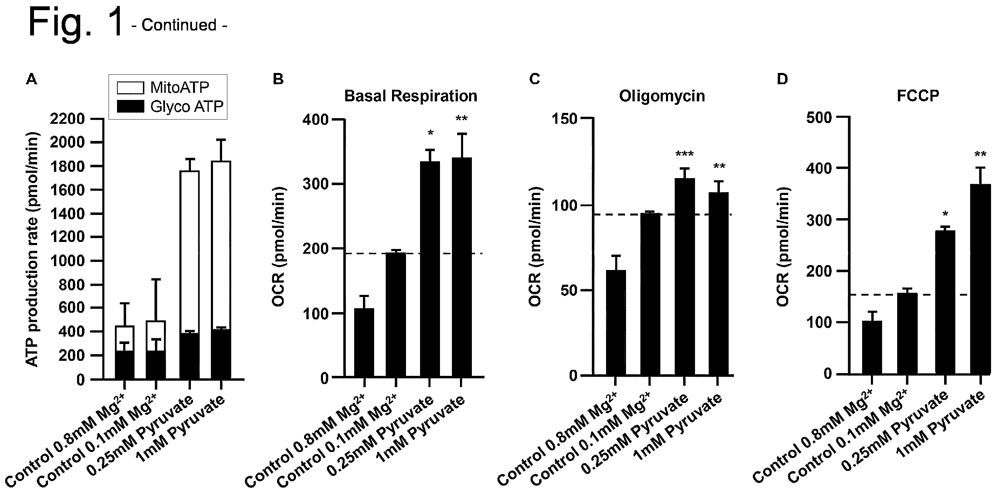

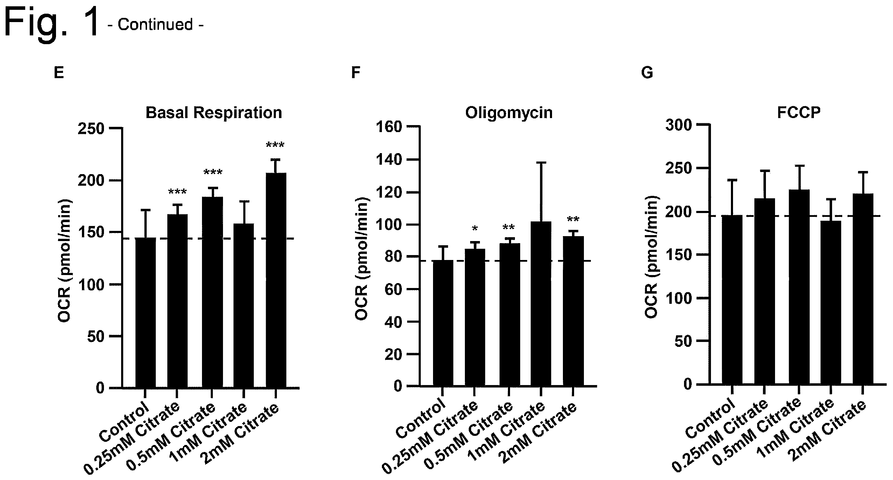

On DIV17, the medium was exchanged for unbuffered Hank's Balanced Salt Solution (HBSS) (a nutrient deprived medium) supplemented with 0.1mM Mg²⁺, 1mM glucose and a concentration range of the test nutrient. The switch from 5mM to 1mM glucose matched the glucose concentrations in the human brain. Meanwhile the switch from high (0.8mM) to low (0.1mM) Mg²⁺ increases the electrical firing frequency of neurons because Mg2+ is an inhibitor of excitatory NMDA-type ionotropic glutamate receptors, which account for most of the neuronal firing in this cell culture model. The increase in neuronal firing caused by incubation in 0.1mM Mg²⁺ increases the energy production of neurons and glial cells as reflected in the increase in basal respiration (Fig. 1B and E). After 1 hour of pre-incubation in the Seahorse medium containing the nutrients, the oxygen consumption rate [OCRs] and extracellular acidification rate [ECARs] of the neuroglial culture were measured in the Seahorse assay.

### Seahorse assay

The oxygen consumption rates (OCRs) and extracellular acidification rates (ECARs) of cells seeded in 96-well plates were monitored in real time at 37°C in unbuffered Hanks Balanced Salt Solution (HBSS). The OCRs represent foremost mitochondrial respiration, and to a minor extent non-mitochondrial respiration. The ECARs relate foremost to the acidification of the medium with lactate produced through glycolysis and secreted by the cells into the medium (Table 1).

During the Seahorse run, both the OCRs and ECARs were measured simultaneously at baseline and following the injection of compounds by the Seahorse instrument (Table 1). HBSS was supplemented with glucose from the start and no glucose was injected through the Seahorse instruments. Measurements were repeated three times at baseline and three times after each compound injection.

**Table 1. Compounds used during Seahorse assay and their effect on oxygen consumption rate (OCR) and extracellular acidification rate (ECAR).**

| **compound** | **Effect on OCR (mitochondrial respiration)** | **Effect on ECAR (lactate acidification - glycolysis)** |
|---|---|---|
| oligomycin | Decrease (proton leak) | Increase |
| FCCP | Increase (max respiratory capacity) | Further increase to max glycolytic capacity |
| Rotenone/antimycin A | Decrease (only non-mitochondrial respiration) | |

### Seahorse - OCR -oligomycin - mitochondrial ATP synthesis

After the initial baseline measurement, oligomycin (OM) was injected. OM blocks mitochondrial ATP synthase (complex V) and therefore decreases mitochondrial respiration. The OCRs after OM injection are due to the mitochondrial proton leak. They enable to determine whether the test nutrients impact the proton leak. The difference in OCRs at baseline and after OM injection is the amount of oxygen used for mitochondrial ATP synthesis at baseline. From this difference in OCR, the mitochondrial ATP production rates are calculated.

### Seahorse - OCR -FCCP - maximal respiratory capacity

After OM, uncoupling agent carbonyl cyanide-p- trifluoromethoxyphenyl-hydrazon [FCCP] was injected which collapses the proton gradient across the inner mitochondrial membrane established during respiration and required for ATP synthesis. The cells attempt to restore the proton gradient following FCCP injection by maximizing respiration. The OCRs measured after FCCP injection therefore represent the maximal respiratory capacity of the mitochondria in the sample and enable to determine whether test nutrients impact the maximal respiratory capacity of the mitochondria.

### Seahorse - OCR - Antimycin A and Rotenone - non-mitochondrial respiration

Finally, Antimycin A , a complex III inhibitor, and Rotenone, a complex I inhibitor, were co-injected, both blocking the respiratory chain at different sites to entirely shut down mitochondrial respiration and determine the non-mitochondrial respiration. The latter is necessary to correct all OCRs and obtain the mitochondrial OCRs.

### Seahorse - ECARs

The ECARs were measured simultaneously at baseline and after OM and FCCP injection. The glycolytic ATP production was calculated from the baseline ECARs. The subsequent injection of OM pushed the cells to maximize their glycolytic ATP production to compensate for the loss in mitochondrial ATP production inflicted by OM. The further addition of FCCP drove mitochondrial respiration to a maximum and this eventually led to a further increase in glycolytic rates providing pyruvate into the TCA cycle to sustain maximal respiration. Therefore, the ECARs after FCCP injection were used to determine the maximum glycolytic capacity of the cells.

### I. Seahorse - low Mg2+ - seizure-like conditions

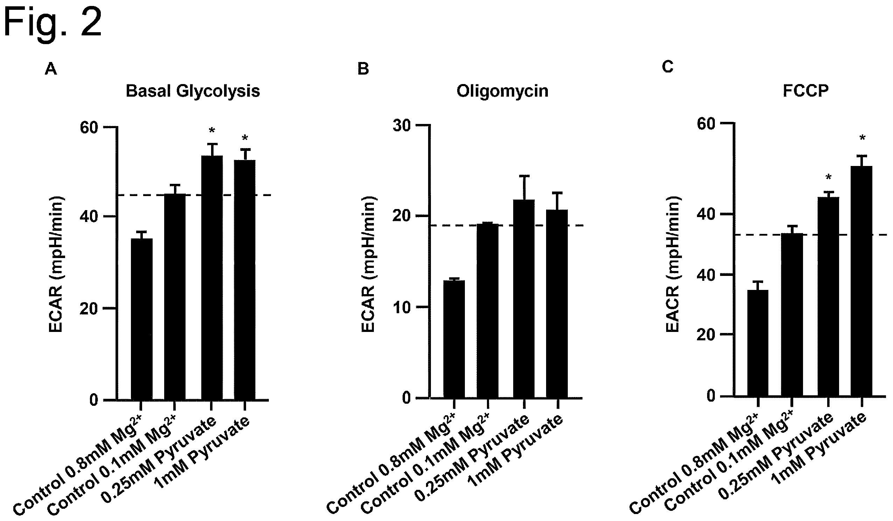

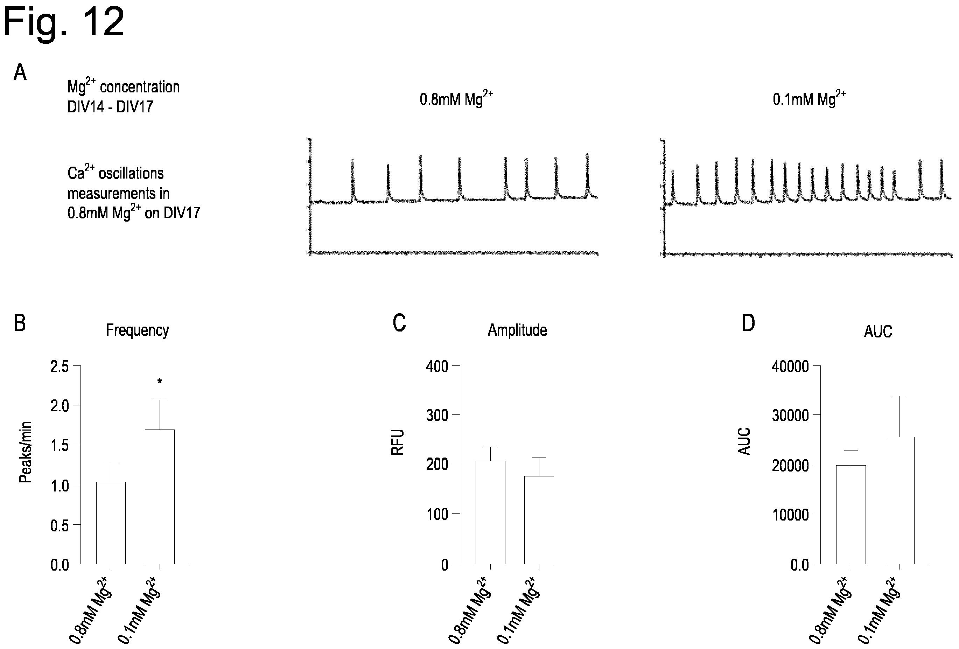

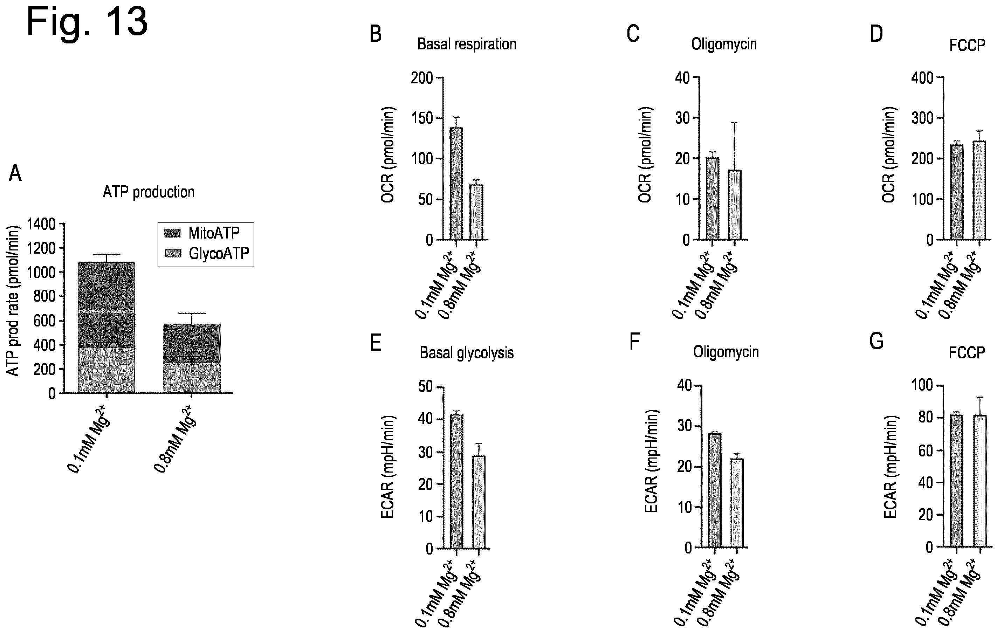

Neuroglial cultures were prepared and assayed from DIV0 until DIV14 as described above. They were switched one hour prior to the Seahorse run to unbuffered Hanks Balanced Salt Solution (HBSS) supplemented with 5mM glucose, 2mM glutamine, 0.1mM or 0.8 mM Mg2+, and 1mM citrate. The switch from high (0.8mM) Mg2+ to low (0.1mM) Mg2+ in the medium increased the firing frequency of neurons (figure 12). The increase in neuronal firing caused by incubation in 0.1mM Mg2+ medium created a seizure-like state and increased the energy demand and production of neurons and glial cells (figure 13). This was reflected by an increase in mitochondrial and glycolytic ATP production (Fig. 13A), an increase in respiration (Fig. 13B) and glycolysis (Fig. 13E) at baseline, and an increase in oligomycin driven glycolytic capacity (Fig. 13F). The switch from high (0.8mM) to low (0.1mM) Mg2+ medium immediately prior to the Seahorse run acutely increased the glycolytic rates by ~10-15% at baseline (Fig. 2A) and by ~30-40% after FCCP injection (Fig 2C).

After 1 hour of pre-incubation in the Seahorse medium containing the test nutrient, the OCRs and ECARs were measured as described for the Seahorse assay. The cells were left in this medium until the end of the run.

### Acute effects of pyruvate under seizure-like conditions

In a first set of experiments, the acute (or immediate) effects of pyruvate were tested in neuroglial cultures acutely switched from high Mg2+ medium to low Mg2+ medium, that is under heightened neuro-excitability or seizure-like conditions. The addition of 0.25mM and 1mM pyruvate to the low Mg2+ medium further increased the total (mitochondrial and glycolytic) ATP production rates by ~400-500% (Fig. 1A), the basal respiration by ~ 30-40% (Fig. 1B) and the FCCP-driven maximal respiratory capacity (Fig. 1D) by ~ 200-250% while modestly augmenting the oligomycin-induced proton leak (Fig. 1C) by ~10-15% compared to low Mg2+ medium not containing pyruvate. Therefore, pyruvate acutely increased basal mitochondrial respiration, basal glycolytic and mitochondrial ATP production and the maximal respiratory capacity of neurons and glial cells under seizure-like conditions.

Moreover, the acute (or immediate) addition of 0.25 mM and 1 mM pyruvate to the low Mg2+ medium increased the glycolytic rates further by ~ 15-20% at baseline (Fig. 2A) and by ~20-30% after FCCP injection (Fig. 2C) compared to low Mg2+ medium and without addition of pyruvate.

Therefore, pyruvate acutely increased basal and maximal glycolytic rates and lactate secretion of neuroglial cell cultures under heightened neuroexcitability or seizure-like conditions.

### Acute effects of citrate under normal and seizure-like conditions

### Resting condition - OCR

In a second set of experiments, the acute or immediate effects of citrate were tested in resting neuroglial cultures incubated in high Mg2+ medium (nutrient-rich DMEM containing 5 mM glucose). The addition of citrate dose-dependently increased the mitochondrial oxygen consumption rates (OCR) at baseline by up to ~25% (Fig. 1E), and the OM-induced proton leak moderately by up to ~10 % (Fig. 1F) while not significantly impacting the FCCP-driven maximal respiratory capacity (Fig. 1G) of the cells compared to incubation in control medium not containing citrate.

### Resting condition - ECAR

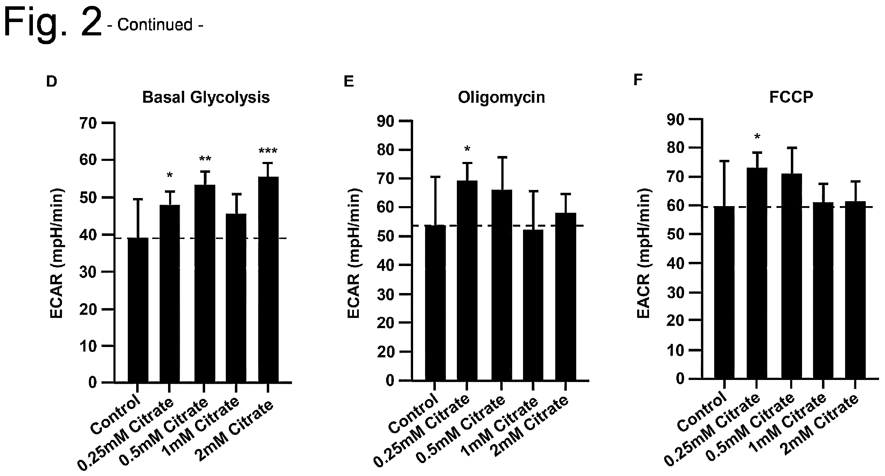

Moreover, the acute (or immediate) addition of citrate dose-dependently increased basal glycolytic rates and lactate secretion by ~25% (Fig. 2D). Furthermore, the acute (or immediate) addition of 0.25 mM citrate increased the OM- and FCCP-induced maximal glycolytic capacity by ~10-15 % (Fig. 2E, F).

Therefore, citric acid provision acutely increased mitochondrial respiration at baseline as well as the basal and maximum glycolytic capacity of neurons and glial cells under resting conditions.

### Seizure-like conditions -OCR

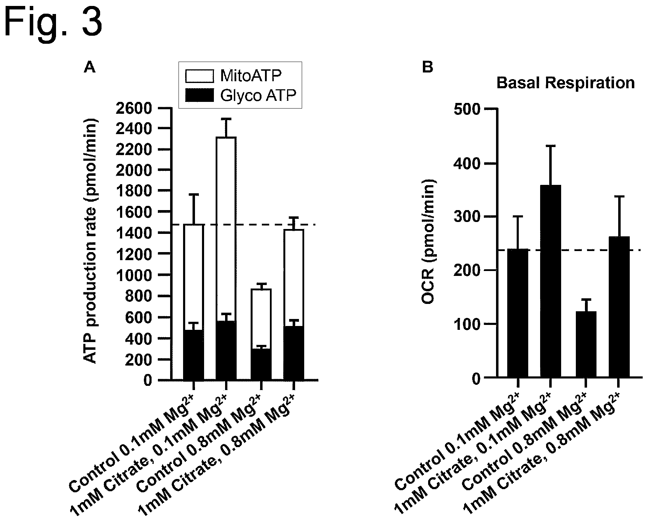

Subsequently, the acute (or immediate) effect of citrate on mitochondrial respiration and glycolysis was also assessed in low Mg2+ medium, that is under heightened neuroexcitability or seizure-like conditions, and compared to incubation in high Mg2+ medium, that is under resting conditions. The addition of 1mM citrate to the medium increased the total (mitochondrial and glycolytic) ATP production by ~ 60% in 0.1mM Mg2+ control medium and by ~ 75% in 0.8mM Mg2+ control medium (Fig. 3A). Furthermore, it increased the basal mitochondrial respiration by ~ 40% in 0.1mM Mg2+ control medium and by ~ 100% in 0.8mM Mg2+ control medium (Fig. 3B). The basal ATP production rates and basal oxygen consumption rates increased when switching the Mg2+ concentration from 0.8 mM to 0.1 mM, consistent with an increased energy demand due to increased neuronal firing. Panel B is corrected for the non-mitochondrial oxygen consumption as determined by addition of Rotenone and Antimycin A. n = 5 repeats/condition.

Hence, citrate acutely increased the total ATP production and mitochondrial respiration of neuroglial cell cultures under resting and heightened neuroexcitability or seizure-like conditions.

### Seizure-like conditions -ECAR

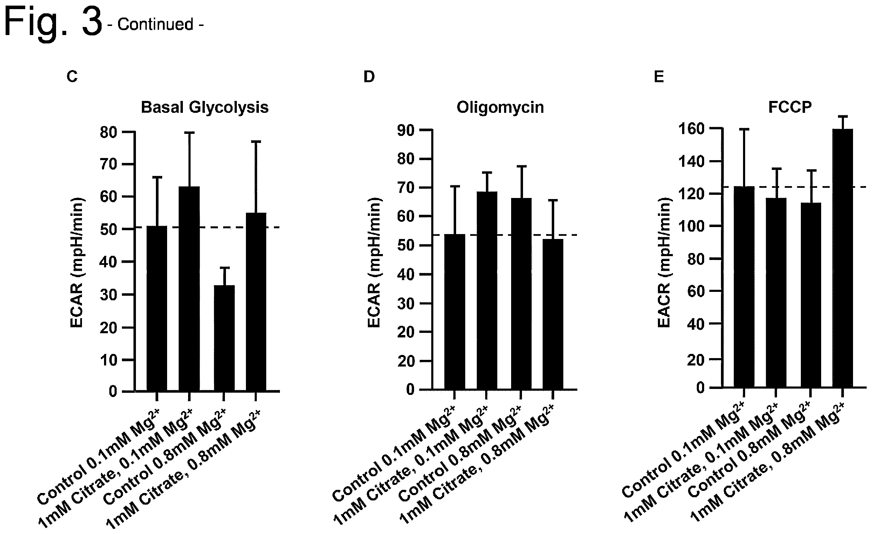

Moreover, the ECAR measurements showed that the addition of 1 mM citrate increased the basal glycolytic rates by ~ 20% in 0.1mM Mg2+ medium and by ~ 70% in 0.8mM Mg2+ medium (Fig. 3C). Furthermore, the addition of 1mM citrate tended to increase the OM-induced glycolytic rates by ~ 15% in 0.1mM Mg2+ medium and by ~ 60% in 0.8mM Mg2+ medium (Fig. 3D). Finally, 1mM citrate also tended to increase the FCCP-driven maximal glycolytic capacity by ~ 35% in 0.8mM Mg2+ control medium (Fig. 3E). Hence, citrate tended to acutely increase the glycolytic rates and lactate secretion of neuroglial cell cultures both under resting and heightened neuroexcitability or seizure-like conditions.

Overall, the results in Fig 3 illustrate that citrate acutely increases basal mitochondrial and glycolytic ATP production and basal mitochondrial respiration both in high (0.8 mM) Mg²⁺ medium and in seizure-inducing low (0.1 mM) Mg²⁺ medium. Moreover, citrate tended to increase the basal and maximal glycolytic rates and lactate secretion, showing that citrate enables neuroglial cells to meet a sudden energy demand arising from an acute electrical hyperstimulation or an epileptic seizure. These results overall indicate that during a seizure, when neurons and glial cells have a greater energy demand, this demand may be better covered by supplementation with pyruvate and citric acid.

The finding that both pyruvate and citrate under resting and heightened neuroexcitability or seizure-like conditions increase glycolysis is very unexpected. Pyruvate is an end-product of glycolysis and its accumulation should decrease glycolytic rates. Meanwhile, citrate is a known physiological inhibitor of glycolysis as it inhibits various tissue- and brain-specific isoforms of the rate-limiting enzyme of the glycolytic pathway called Phosphofructokinase 1 (PFK1) at concentrations above 100µM.

### II. 24-hour effect of citrate under resting conditions

Further, the effects of a 24-hour incubation with citrate on glycolysis, mitochondrial respiration and total (glycolytic and mitochondrial) energy (ATP) production under resting conditions was assessed. Neuroglial cell cultures were grown in Neurobasal medium (NBM) supplemented with B27, high (25mM) glucose, and high (0.8mM) Mg2+ from DIV0 until DIV14. They were switched to NBM supplemented with B27, low (5mM) glucose, and high (0.8mM) Mg²⁺ from DIV14 until DIV16. On DIV16, the medium was exchanged for DMEM (which is, in contrast to HBSS, not a nutrient-deprived medium) containing low (5mM) glucose, high (0.8 mM) Mg²⁺ and 500 µM or 1 mM citrate. On DIV17, one hour prior to the Seahorse run, the medium was replaced with unbuffered DMEM containing low (5mM) glucose, high (0.8 mM) Mg²⁺ and 500µM or 1mM citrate. The cells were left in this medium until the end of the run. Five repeats per condition were done.

### Citrate - OCR

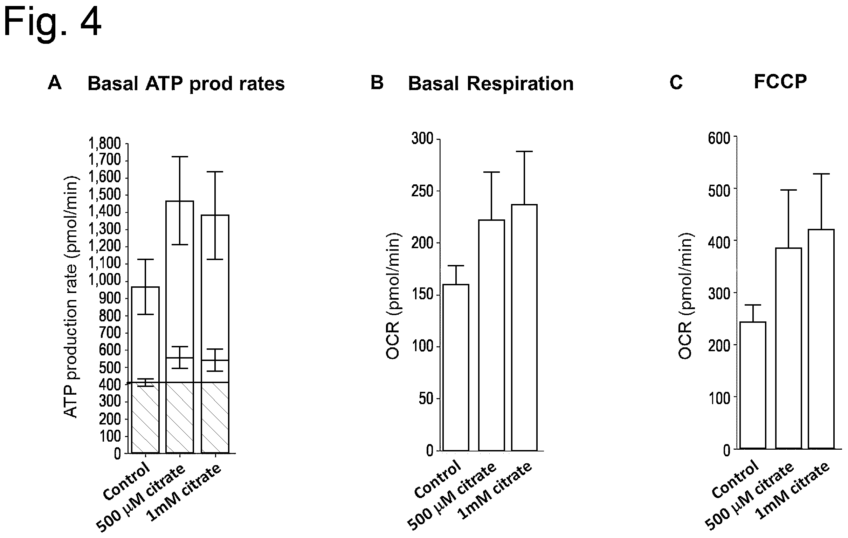

The addition of 500µM and 1mM citrate to the medium increased the basal mitochondrial and glycolytic ATP production by ~ 45% compared to control medium (Fig 4A). Furthermore, it increased the basal mitochondrial respiration by ~ 60% compared to control medium (Fig. 4B) and the FCCP-induced maximal mitochondrial respiratory capacity by ~ 60-70% compared to control medium (Fig. 4C). Hence, a long-term 24-hour incubation with citrate increased the total ATP production, the baseline and maximal mitochondrial respiration of resting neuroglial cell cultures. Panels B and C are corrected for the non-mitochondrial oxygen consumption determined by addition of Rotenone and Antimycin A.

### Citrate - ECAR

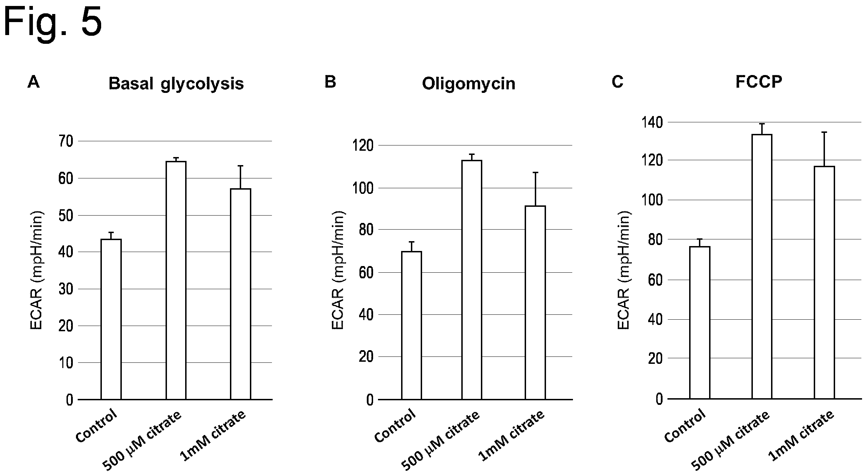

The ECAR measurements concurrent to the OCR measurements showed that the addition of 500µM and 1mM citrate to the medium increased the basal glycolytic rates by ~ 45% and ~20%, respectively, compared to control medium (Fig 5A). Furthermore, it increased the OM-induced increase in glycolytic rates by ~ 70% and ~30%, respectively, compared to control medium (Fig 5B). Finally, it increased the FCCP-induced maximal glycolytic capacity by ~75% and ~ 50% compared to control medium (Fig 5C). Hence, a long-term 24-hour incubation with citrate increased basal and maximal glycolytic rates and lactate secretion of resting neuroglial cell cultures.

The 24-hour incubation in citrate thus increased the basal (glycolytic and mitochondrial) ATP production (figure 4A), the basal and maximal mitochondrial respiration (figure 4B, C), the basal and maximal glycolytic rates and lactate secretion (figure 5) under resting conditions.

The results illustrated in figures 4 and 5 evidence that during a seizure, when neurons and glial cells have a greater energy demand, this demand may be better covered by supplementation with pyruvate and citrate. In addition, the effect of pyruvate is independent of other nutrients as its effect is seen in a nutrient poor HBSS medium, whereas the single effect of citrate was seen only in nutrient-rich DMEM, indicating that the effect of citrate is dependent on the presence of additional nutrients.

### III. Acute effects of citrate and pyruvate under seizure- and ketogenic diet-like conditions

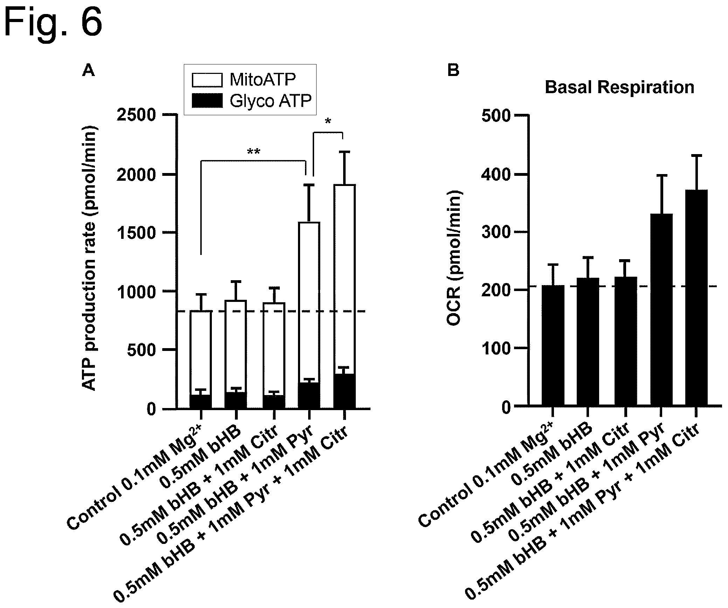

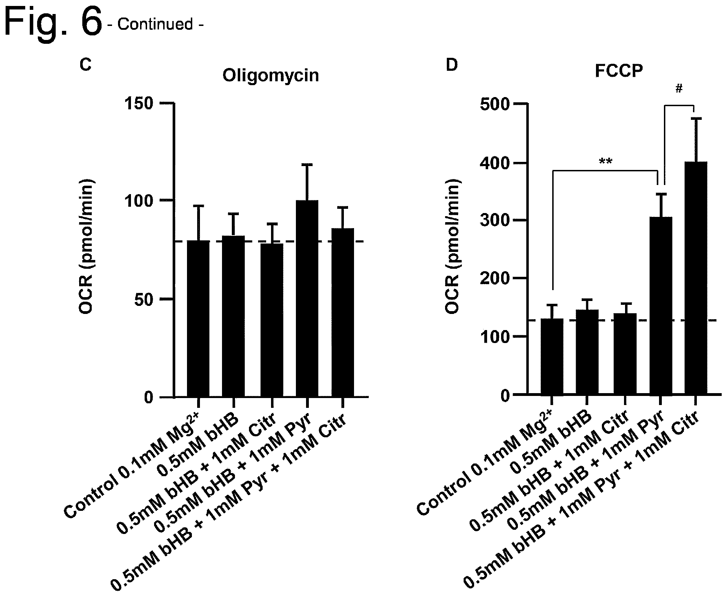

To assess the effect of citrate and pyruvate under seizure- and ketogenic diet-like conditions a batch of cells was switched one hour prior to the Seahorse run to HBSS medium (which is a nutrient deprived culture medium) containing low (1 mM) glucose, 500 µM bHB and low (0.1 mM) Mg²⁺, 1mM citrate or 1mM pyruvate (Fig. 6). The ketone β-hydroxybutyrate (bHB) was included during the Seahorse run at a concentration of 500µM to mimic the nutrient environment of a ketogenic diet. This bHB concentration corresponds to the circulating bHB concentration of a mild ketogenic diet such as the Modified Atkins Diet.

For the Seahorse assay that was run under seizure- and ketogenic diet like-conditions the cells were then incubated in HBSS medium containing low (0.1mM) Mg²⁺ and one of the following additions:
i)1mM glucose (control medium),
ii) 500µM bHB,
iii) 500µM bHB plus 1mM citrate,
iv) 500µM bHB plus 1mM pyruvate or
v) 500µM bHB with 1mM citrate plus 1mM pyruvate.

The addition of 500µM bHB did not impact mitochondrial respiration and ATP production at baseline or the FCCP-induced maximal mitochondrial respiratory capacity compared to control medium (Fig. 6 A, B, D).

### OCR - bHB and pyruvate or citrate

The addition of 500 µM bHB and 1mM pyruvate to the medium increased the total (and specifically the glycolytic) ATP production by ~ 100% (** P < 0.01) compared to control medium. Furthermore, it increased the basal respiration by ~ 50% (Fig. 6B) and the FCCP-driven maximal respiratory capacity by ~ 100% (** P < 0.01; Fig. 6D) while not impacting the oligomycin-induced proton leak (Fig. 6C) compared to control medium.

The addition of 500µM bHB and 1mM citrate to the medium did not alter the total, mitochondrial or glycolytic ATP production (Fig. 4A), the basal mitochondrial respiration (Fig.6B) or the FCCP-induced maximal mitochondrial respiratory capacity (Fig. 4D).

### OCR - combination of bHB, pyruvate and citrate

However, the addition of 500µM bHB and 1mM pyruvate and 1 mM citrate yielded an additional gain in total and specifically glycolytic ATP production (* P < 0.05; Fig. 6A) and in FCCP-driven maximal respiratory capacity (# P = 0.09; Fig. 6D) compared to the effect (apparent as the gain above the threshold value (dashed horizontal line)) of the addition of 500µM bHB and 1mM pyruvate. Hence, citrate potentiated in a more then additive manner the pyruvate-induced gain in ATP production and respiratory capacity of neurons and glial cells under seizure- and ketogenic diet-like conditions. Statistical analysis was done by non-parametric Mann-Whitney test with 5 repeats per condition. Fig. 6 B-D are corrected for the non-mitochondrial oxygen consumption as determined by addition of Rotenone and Antimycin A.

Overall, the data in Fig 6 shows that pyruvate acutely increases basal (mitochondrial and glycolytic) ATP production, basal mitochondrial respiration and the FCCP-induced maximal mitochondrial respiratory capacity of neuroglial cells under seizure- and ketogenic diet-like conditions. Moreover, the data in Fig. 6 show that citrate acutely potentiates in a more than additive manner the effects of pyruvate on ATP production and mitochondrial respiration under seizure- and ketogenic conditions.

### ECAR measurements

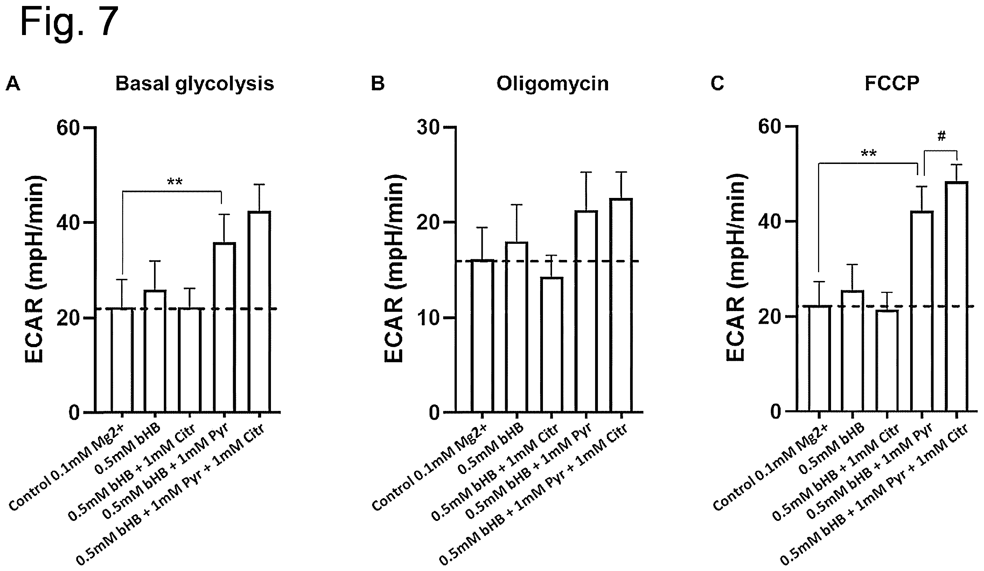

ECAR measurements were obtained concurrent to the OCR measurements described above and shown in figure 6. For the Seahorse run the cells were as described above incubated in HBSS medium containing low (0.1mM) Mg2+, 1mM glucose, 500 µM bHB and 1mM citrate, or 1mM pyruvate or 1mM citrate plus 1mM pyruvate. These incubations were compared to incubation in the same medium without the addition of bHB, citrate or pyruvate ("Control 0.1mM Mg²⁺"). The addition of 500 µM bHB to the medium did not alter baseline or maximal glycolytic rates (Fig. 7A, C) or baseline glycolytic ATP production (figure 6A) compared to control medium.

### ECAR - bHB and pyruvate

The addition of 500 µM bHB and 1mM pyruvate to the medium increased the basal glycolytic ATP production by ~ 100% compared to control medium (** P < 0.01; Fig 6A). Furthermore, it increased basal glycolysis by ~ 50% (** P < 0.01; Fig 7A) and the FCCP-driven maximal glycolytic capacity by ~ 100% (** P < 0.01; Fig. 7C) compared to control medium.

### ECAR - bHB and citrate

The addition of 500µM bHB and 1mM citrate did not alter basal glycolytic ATP production (Fig 6A), basal glycolysis (Fig 7A) or maximal glycolytic capacity (Fig 7C) compared to control medium.

### ECAR combination of bHB, pyruvate and citrate

However, adding 1mM citrate on top of 500 µM bHB, and 1mM pyruvate to the medium yielded an additional gain in basal glycolytic ATP production (* P < 0.05; Fig 7A) and FCCP-driven maximal glycolytic capacity (# P = 0.056; Fig 7C) compared to addition of 500 µM bHB and 1mM pyruvate. Hence, citrate more than additionally potentiated the gain in basal glycolytic ATP production and maximal glycolytic capacity obtained with pyruvate in neurons and glial cells under seizure- and ketogenic diet-like condition.

In summary, these results show that pyruvate increases glycolysis, respiration, and ATP production in neuroglial cells under seizure- and ketogenic diet-like conditions and that this effect is potentiated by citrate. Therefore, providing pyruvate alone or in combination with citrate helps neurons and glial cells to meet the energy demand imposed on them by epileptic seizures and to remain viable. By improving cell viability, the progressive transformation of a normal neuronal network into an epileptic network with repeated seizures is delayed and the clinical worsening of an epileptic disorder in terms of seizure frequency, seizure severity, cognitive and behavioral impairment is improved.

### IV. Long-term effects of citrate and pyruvate in combination with further nutrients under seizure- and ketogenic diet-like conditions

Subsequently, the chronic or long-term effects of pyruvate and citrate in combination with other nutrients on glycolysis, mitochondrial respiration, and ATP production in primary rat neuroglial cell cultures under seizure- and ketogenic diet-like conditions were assessed.

The neuroglial cell cultures were grown in Neurobasal medium (NBM) containing B27, high (25mM) glucose, and high (0.8 mM) Mg²⁺ from DIV0 until DIV14. They were then switched to a custom NBM containing B27, low (1mM) glucose, 500 µM b-hydroxybutyrate (bHB) and low (0.1 mM) Mg2+ from DIV14 until DIV16. The lowering of the glucose concentration and the addition of bHB creates the nutrient conditions of a ketogenic diet. Meanwhile the lowering of the Mg2+ concentration increases the neuronal firing rates creating a seizure-like state (Fig. 12) and increases the energy demand and production of the cells (Fig. 13). Furthermore, the custom NBM was supplemented with test nutrients from DIV14 until DIV16 (Table 2). On DIV16, one hour prior to the Seahorse run, the medium was exchanged for Hank's Balanced Salt Solution (HBSS) containing 1 mM glucose, low (0.1 mM) Mg²⁺, 500 µM bHB and the same test nutrients they were pre-exposed to from DIV14 until DIV16. The cells were left in this medium until the end of the run. Six repeats per condition were performed.

**Table 2. Test nutrients used in the low glucose phase and Seahorse run. X means not supplemented.**

| | **Mg²⁺ (mM)** | **Leu (µM)** | **DHA (µM)** | **EPA (µM)** | **Citr (µM)** | **Pyr (µM)** | **bHB (µM)** | **C4 (µM)** | **NR (µM)** | **NA (µM)** | **NAM (µM)** |
|---|---|---|---|---|---|---|---|---|---|---|---|
| high Leu, high NA | 0.8 | 800 | X | X | X | X | X | X | X | 33 | X |
| high Leu, high NA | 0.1 | 800 | X | X | X | X | X | X | X | 33 | X |
| Low Leu, low NA | 0.1 | 40 | X | X | X | X | X | X | X | 1.65 | X |
| no test nutrients | 0.1 | X | X | X | X | X | X | X | X | X | X |
| Full combi | 0.1 | 400 | 2.5 | 2.5 | 250 | 250 | 500 | 10 | 10 | X | X |
| Full combi no bHB | 0.1 | 400 | 2.5 | 2.5 | 250 | 250 | 500 | X | 10 | X | X |
| Full combi no ω3 PUFAs | 0.1 | 400 | X | X | 250 | 250 | 500 | 10 | 10 | X | X |
| Full combi no citr +pyr | 0.1 | 400 | 2.5 | 2.5 | X | X | 500 | 10 | 10 | X | X |
| Full combi, no Leu | 0.1 | X | 2.5 | 2.5 | 250 | 250 | 500 | 10 | 10 | X | X |
| Full combi with niacin (NA+NAM), no NR | 0.1 | 400 | 2.5 | 2.5 | 250 | 250 | 500 | 10 | X | 5 | 5 |

Regular neurobasal medium (NBM) is devoid of most test nutrients, except leucine and vitamin B3 which is present in the form of nicotinic acid (NA). Therefore, a custom NBM devoid of leucine and NA was used during the low glucose phase from DIV14 until DIV16 to i) add leucine and NA at desired concentrations, ii) to substitute NA for the other B3 members Niacin (combination of NA and Nicotinamide (NAM)) and Nicotinamide riboside (NR. The following nutrients were tested in combination (denoted full combination) using the concentrations shown in table 1: the anaplerotics citrate (Citr) and pyruvate (Pyr); leucine (Leu); the ω3 polyunsaturated fatty acids (w3- PUFAs) docosahexaenoic acid (DHA) and eicosapentaenoic acid (EPA); the short chain fatty acid butyrate (C4); and the vitamin B3 member NR. To assess the individual contributions of these nutrients, the full combination was also tested without anaplerotics (Citr + Pyr), or Leu, or butyrate (C4), or ω3-polyunsaturated fatty acids (DHA + EPA), or with Niacin (NA + NAM in a 1:1 ratio) in exchange for NR. DHA and EPA were coupled to bovine serum albumin (BSA) to solubilize them in the medium and facilitate their uptake by the cells.

Since the Leu and B3 (NA) concentration in the regular NBM are much higher than in human blood, they were applied at physiological concentrations. Likewise, all other nutrients in the full combination were applied at normal circulating concentrations. To exclude an impact of reducing the Leu and NA concentrations in NBM on cell viability, we compared cells fed with high levels of Leu and NA in regular NBM, to cells fed with low levels of Leu and NA in custom NBM and to cells not fed with Leu and NA in NBM.

### OCR under seizure- and ketogenic diet-like conditions

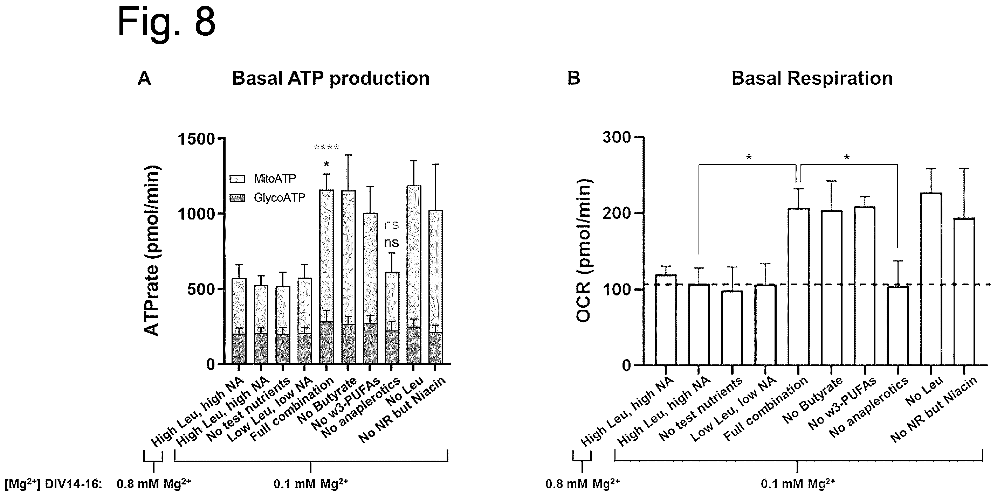

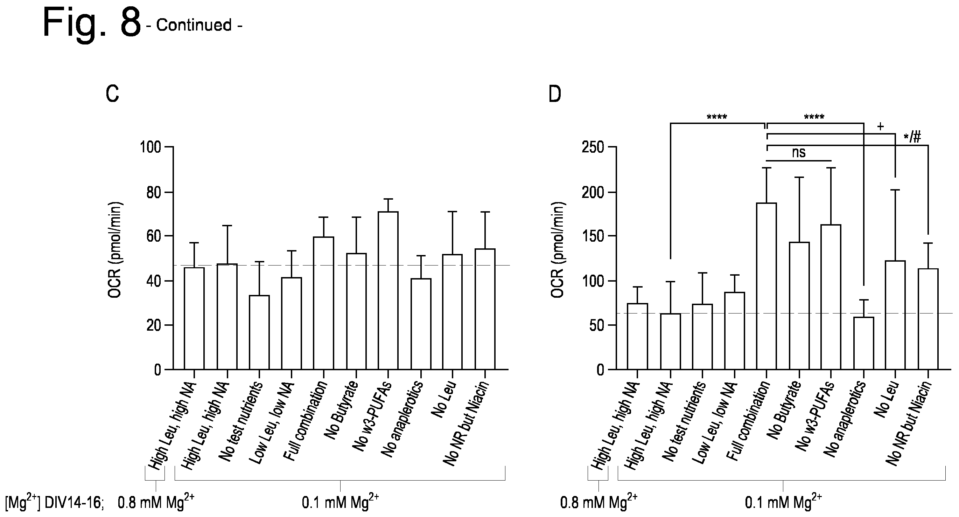

In neuroglial cell cultures under chronic seizure- and ketogenic diet-like conditions the full combination of test nutrients consisting of anaplerotics according to table 1 with 250 µM pyruvate and 250 µM citrate, w3-PUFAs (2.5 µM DHA and 2.5µM EPA), butyrate (4 µM), Leu (400µM), and NR (10 µM) significantly raised the basal (mitochondrial and glycolytic) ATP production and respiration by ~100% (Fig. 8A, B), and the FCCP-induced maximal respiratory capacity by ~200% (Fig 8D) while not impacting the oligomycin-induced proton leak (Fig 8C) compared to regular NBM containing 800 µM Leu and 33 µM NA (high Leu, high NA condition).

These responses were mainly driven by the anaplerotics since the increases in basal ATP production (Fig 8A), basal respiration (Fig. 8B) and maximal respiratory capacity (Fig 8D) were no longer seen in the absence of the anaplerotics in the full combination.

The FCCP-induced maximal respiratory capacity achieved with the full combination was reduced by ~ 30-40% with the exchange of 10 µM NR for 10 µM Niacin (* P = 0.051) and the absence of Leu (+ P = 0.12) compared to the full combination containing these ingredients (Fig 8D). The FCCP-induced maximal respiratory capacity was the same when the medium (HBSS) has no Leu or B3 (so-called **no test nutrient condition)** or does have Leu or B3 (high Leu, high NA condition; Low Leu, low NA condition). Therefore, neither classical Vitamin B3 nor Leu have an impact by themselves on maximal respiration. However, when Leu is removed from the full combination or when NR is exchanged for Niacin in the full combination, then surprisingly a significant drop in maximal respiration is seen relative to the full combination. This would not be expected since maximal respiration is not affected by their presence or absence at either low or high concentration in the HBSS medium. Therefore, Leu and NR both have a surprising synergistic effect in the full combination. This illustrates that both leucine and NR potentiate the gain in maximal respiratory capacity achieved with pyruvate and citrate in the full combination of nutrients. Meanwhile the absence of butyrate and w3-PUFAs in the full combination did not alter mitochondrial respiration or ATP production (Figs. 8A, B, D). Hence, the full response to the full combination was driven by the anaplerotics with some potentiation by Leu and NR.

Depriving NBM of Leu and Niacin (low leu, low NA condition, no test nutrient condition) had no significant impact on respiration or ATP production. **** P < 0.0001; * P = 0.051; + P = 0.12 versus regular NBM (high Leu, high NA condition) by non-parametric One-way ANOVA followed by Dunnett's multiple comparisons test; # P < 0.01 versus regular NBM (high Leu, high NA condition) by t test; Panels B-D are corrected for the non-mitochondrial oxygen consumption determined by the addition of Rotenone and Antimycin A.

In conclusion, pyruvate and citrate in the full combination increased mitochondrial ATP production and respiration at baseline, as well as the maximal respiratory capacity of neurons and glial cells under seizure- and ketogenic diet-like conditions. Moreover, it was found that both leucine and NR (in exchange for Niacin (NA plus NAM)) potentiated the gain in maximal respiratory capacity achieved with pyruvate and citrate in the full combination.

### ECAR seizure- and ketogenic diet-like conditions

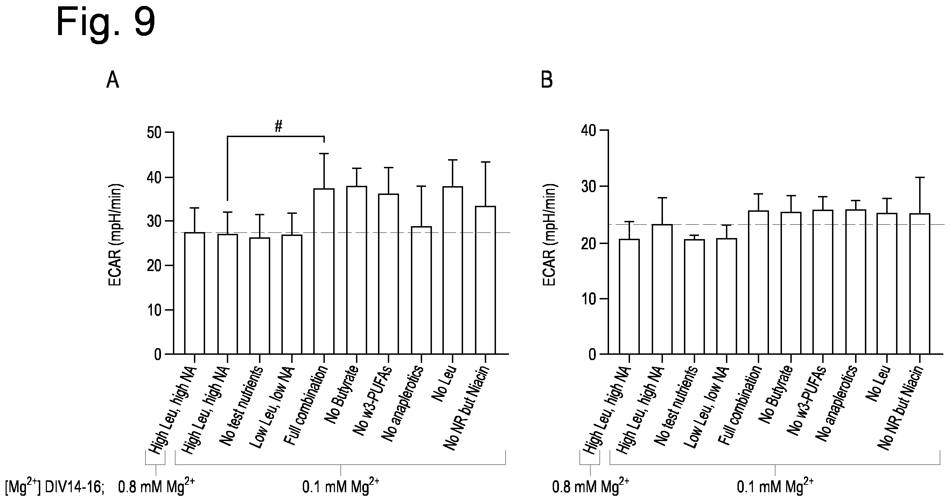

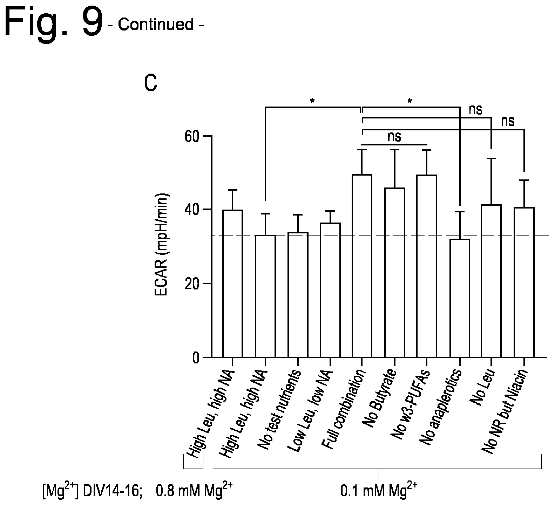

ECAR measurements concurrent to the OCR measurements shown in figure 8 were also performed under chronic seizure- and ketogenic diet-like conditions with the different combinations of test nutrients according to Table 1. The full combination of test nutrients significantly raised the glycolytic rates at baseline by ~25% (Fig. 9A), and after maximal stimulation with FCCP by ~30% (Fig. 9C) compared to regular NBM containing 800 µM Leu and 33µM NA (high Leu, high NA condition). This response was driven by the anaplerotics pyruvate and citrate since the increases in basal glycolysis (Fig. 9A) or maximal glycolytic capacity (Fig 9C) were no longer seen in the absence of the anaplerotics in the full combination.

Meanwhile, the absence of butyrate, w3-PUFAs, Leu, or the exchange of NR for Niacin in the full combination caused no significant change in glycolytic activities. Hence, pyruvate and citrate in the full nutrient combination mediated the increase in baseline and maximal glycolytic activity of neurons and glial cells under chronic seizure- and ketogenic diet-like conditions.

In summary the data presented in Fig 8 and 9 demonstrate that a combination of pyruvate and citrate increases glycolysis, respiration and ATP production in neuroglial cells under seizure- and ketogenic diet-like conditions and that Leu and NR potentiate these effects. Therefore, a nutrient combination containing pyruvate, citrate, Leu and NR helps neurons and glial cells to cope with the energy demand of repeated epileptic seizures and to remain viable. This nutrient combination would therefore delay the transformation of a normal neuronal network into an epileptic network with repeated seizures and improve the clinical course of an epilepsy in terms of seizure frequency, seizure severity, cognitive and behavioral impairments.

### V. Effect pyruvate and citrate on cell death protection

To determine whether pyruvate and citrate protect neurons and glial cells from cell death induced by chronic electrical hyperstimulation. To assess protection from cell death induced by repeated hyperstimulation, primary mouse neuroglial cultures were assayed. Neuroglial cell cultures were prepared and grown until DIV14 as described above. From DIV14 until DIV17 they were switched to NBM containing B27, low (2.5mM) glucose, and low (0.1 mM) Mg²⁺ or high (0.8 mM) Mg²⁺. Incubation in low (0.1 mM) Mg²⁺ medium increases the neuronal firing rates, thus creating a state of chronic hyperstimulation. The test nutrients (pyruvate or citrate) were supplied through the medium from DIV14 until DIV17. On DIV17, the cell nuclei were stained with the Hoechst dye and co-immunolabelled for NeuN, a protein found only in the nuclei of neurons. The neuronal and non-neuronal cell nuclei were scored for nuclear shrinkage, a hallmark of cell death, by high content imaging.

### Cell death assessment

To assess cell death by high content imaging the cells were fixed on DIV17 in 1% formaldehyde, permeabilized with ethanol and incubated with a rabbit polyclonal IgG antibody against NeuN, a neuron-specific protein localized to the nucleus, followed by detection with a secondary anti-rabbit IgG antibody linked to a red fluorescent label. Neuronal and non-neuronal cell nuclei were then counterstained with the blue fluorescent nuclear dye Hoechst. The nuclei were counted by a high content imager based on signal intensity and size. A cut-off value was applied to exclude objects of low signal intensity considered noise. Then, cut-off values were applied to exclude objects with an area below 30 µM²considered cell debris and objects with an area above 300 µM² considered cell clumps. A boundary of 60 µM² was set to distinguish between nuclei from alive and dead cells. Objects areas of 30 µM² to 60 µM² were counted as nuclei from dead cells, while object areas of 60 µM² to 300 µM²were counted as nuclei from alive cells. The counting was done at 20-fold magnification on a fixed number of optical fields per well. The counts of nuclei from dead neurons or dead cells were related to the number of alive neurons or alive cells, respectively, in the same well.

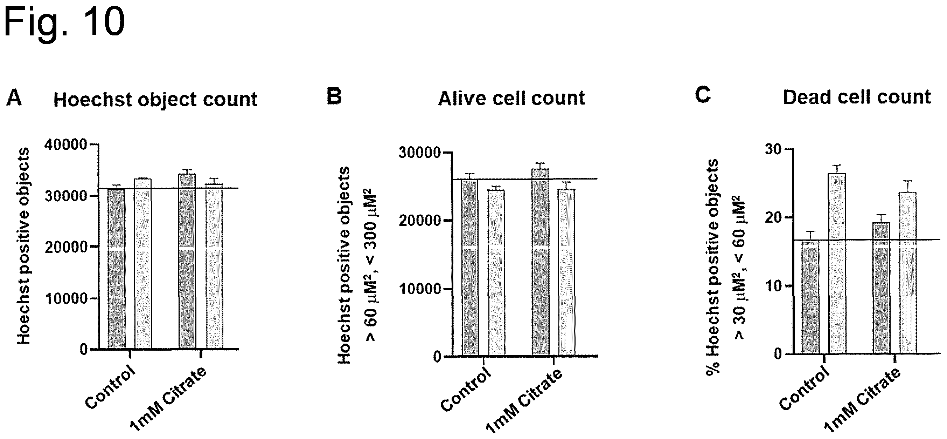

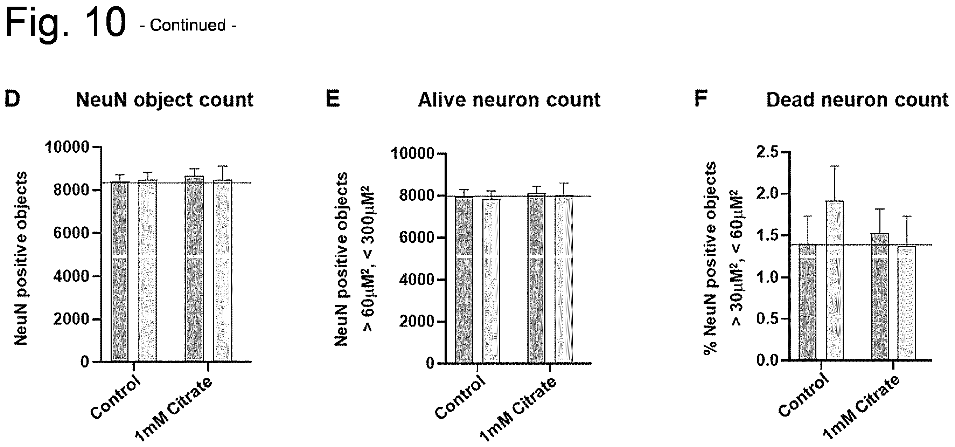

Neuroglial cell cultures were supplemented with 1mM citrate from DIV14 until DIV17. On DIV17, total cell death and neuron-specific cell death were quantified by high content imaging. The total number of Hoechst positive objects or NeuN positive objects prior to applying cut-off values for the signal intensity and the area size was equal between the different groups (Fig 10 A, D).

Chronic hyperstimulation from DIV14 until DIV17 increased the dead cell counts by ~ 7% from ~ 18% in 0.8mM Mg2+ medium to ~ 25% in 0.1mM Mg²⁺ medium (Fig 10C). The number of alive cells was not or only marginally reduced (Fig 10B), possibly due to compensatory cell proliferation.

### Effect of citrate on cell death

Citrate supplementation led to a smaller increase in dead cell counts of ~ 4% from ~ 19% in high Mg²⁺ medium to ~ 23% in low Mg2+ medium (Fig 10C) while not impacting the alive cell count (Fig 10B). Chronic hyperstimulation increased the dead neuron count by ~0.4% from ~1.4% in high Mg²⁺ medium to ~1.8% in low Mg2+ medium (Fig. 10F). Citrate supplementation entirely blocked the rise in neuron death from ~1.5% in high Mg²⁺ medium to ~1.4% in low Mg²⁺ medium (Fig 10F). The total number of alive neurons did not significantly differ between low and high Mg²⁺ medium (Fig 10E). In summary, citrate protected neurons and glial cells from excitotoxic cell death induced by chronic hyperstimulation.

### Effect of pyruvate on cell death

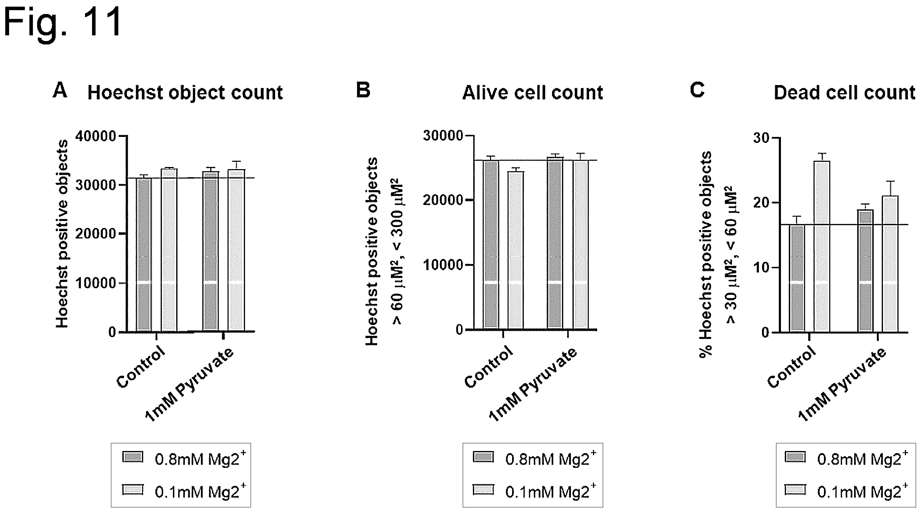

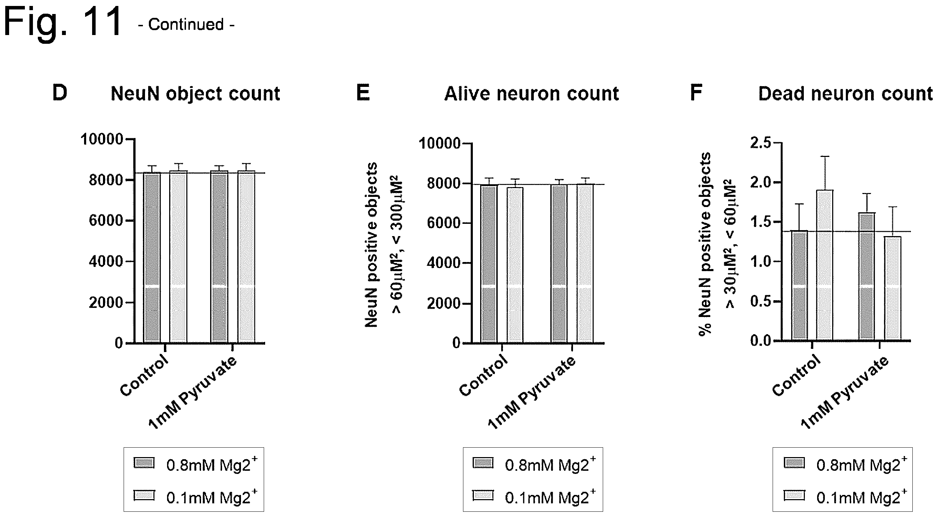

The same assessment was done with neuroglial cells supplemented with 1mM pyruvate. Also here, the total number of Hoechst positive objects or NeuN positive objects prior to applying cut-off values for the signal intensity or the area size was equal between the different groups (Fig 11 A, D). Chronic hyperstimulation from DIV14 until DIV17 increased the dead cell counts by ~ 7% from ~ 18% in 0.8mM Mg2+ medium to ~ 26% in 0.1mM Mg²⁺ medium (Fig. 11C). The number of alive cells was not or only marginally reduced (Fig. 11B). Pyruvate supplementation led to a markedly smaller increase in dead cell counts of ~ 1% from ~ 19% in high Mg²⁺ medium to ~ 20% in low Mg²⁺ medium (Fig 11C) while not impacting the alive cell count (Fig. 11B). Chronic hyperstimulation increased the dead neuron count by ~0.4% from ~1.4% in 0.8mM Mg²⁺ medium to ~1.8% in 0.1mM Mg²⁺ medium (Fig. 11F). Pyruvate supplementation blocked and even reduced neuron death from ~1.5% in high Mg²⁺ medium to ~1.4% in low Mg²⁺ medium (Fig 11F). The total number of alive neurons did not significantly differ between low and high Mg2+ medium (Fig 11E).

In summary, both citrate and pyruvate protected neurons and glial cells from excitotoxic cell death induced by chronic hyperexcitation.

### VI. Network excitability

It was then confirmed that switching neuroglial cell cultures from a high (0.8mM) Mg²⁺ medium to a low (0.1mM) Mg²⁺ medium electrically stimulates the neuronal network, leading to network reorganization and greater network excitability. Electrical stimulation of neurons leads to Ca²⁺ influx into the neurons where Ca²⁺ triggers the synaptic release of neurotransmitters for the propagation of action potentials. Immediately after its influx, Ca²⁺ is exported out of the cell. The influx and efflux of Ca²⁺ produces oscillations. The sum of all Ca²⁺ oscillations in the cell culture is a measure of the electrical activity of the whole neuronal network. With repeating seizures, electrical signals are relayed more frequently and to more neurons through the network. This is due to the network reorganization that is driven with each seizure and facilitates the generation, transmission and ultimately synchronization of electrical signals between neurons. It manifests as an increase in the frequency and/or amplitude of Ca2+ oscillations. To determine such changes in network excitability, we monitored the Ca²⁺ oscillations with Ca²⁺ sensing dyes using a previously published assay (Pacico N et al. PLOS ONE 2012).

Neurons and glial cells were isolated from embryonic day 18 mouse cortices and seeded in 96-well plates. They were then grown from DIV0 until DIV14 in neurobasal medium (NBM) containing B27, 25mM glucose and 0.8mM Mg²⁺. From DIV14 until DIV17, they were switched to NBM containing B27, 2.5mM glucose and either low excitatory 0.1mM Mg²⁺ or high non-excitatory0.8mM Mg²⁺. On DIV17, one hour prior to the measurement, the cells were switched to HBSS containing a Ca²⁺ sensitive fluorescent dye, 5mM glucose, and 0.8mM Mg²⁺ (corresponding to resting conditions). The cells were left in this medium until the end of the measurement.

Neuroglial cell cultures that had been chronically pre-exposed from DIV14 until DIV17 to low excitatory (0.1mM) Mg²⁺ medium showed a significant increase in the frequency (* P < 0.05; Fig. 12A, B) and no change in the amplitude (Fig 12C) of Ca²⁺ oscillations compared to neuroglial cultures that had been pre-exposed to high non-excitatory (0.8mM) Mg²⁺ medium from DIV14 until DIV17. Furthermore, chronic pre-exposure to low Mg²⁺ medium tended to increase the Area Under the Curve (AUC) of the Ca²⁺ oscillations (Fig 12D) due to the frequency increase, indicating an increase in the total amount of Ca²⁺ flowing in and out of the cells. Hence, chronic exposure of neuroglial cell cultures to a low (0.1mM) Mg²⁺ medium led to a permanent increase in neuronal network excitability and activity.

To assess the acute effect of neural excitation on the energy demand neuroglial cell cultures grown in high non-excitatory at 0.8 mM Mg²⁺ medium was acutely exposed to low excitatory Mg²⁺ medium (0.1 mM). An increase in basal (mitochondrial and glycolytic) ATP production (Fig 13A), basal respiration (Fig 13B), and basal glycolysis (Fig 13E) was observed, evidencing a net increase in energy production. The FCCP-induced maximal respiratory capacity (Fig 13D) and maximal glycolytic capacity (Fig 13G) were not altered by acute incubation in low Mg²⁺ medium. Hence, the switch from a high-excitatory Mg²⁺ medium to a low-excitatory Mg²⁺ medium acutely increases baseline glycolytic and mitochondrial energy production of neurons and glial cells.

## Claims

1. A neuroprotective composition comprising therapeutically effective amounts of a combination of pyruvate and citrate, for use in the treatment and/or prevention of neuronal damage in a subject suffering from or at risk of seizures.

2. Neuroprotective composition for use according to claim 1 wherein the treatment and/or prevention of neuronal damage includes the prevention and/or treatment of neuroinflammation, the prevention and/or delay of the formation of epileptiform circuits in the brain.

3. Neuroprotective composition for use according to claims 1 and 2 wherein the treatment and/or prevention of neuronal damage involves an increase in metabolic activity, preferably in glycolysis and mitochondrial oxygen consumption rate of neurons and glial cells in the brain.

4. Neuroprotective composition for use according to the preceding claims wherein the treatment and/or prevention of neuronal damage includes a reduction of excitotoxity associated with or following seizures.

5. Neuroprotective composition for use according to the preceding claims wherein the subject suffering from or at risk of seizures is a subject suffering from stroke, traumatic brain injury, migraine, epilepsy, Alzheimer's disease, Parkinson's disease, or conditions impairing neuronal and glial energy metabolism including Glucose transporter type 1 (Glut1) deficiency, Pyruvate dehydrogenase (PDH) deficiency, glycogen storage diseases and mitochondrial diseases.

6. Neuroprotective composition for use according to the preceding claims, wherein the composition further comprises one or more compounds selected from leucine and nicotinamide riboside.

7. Neuroprotective composition for use according to the preceding claims wherein the subject suffering from or at risk of seizures is adhering to a ketogenic diet, preferably a ketogenic diet with a ketogenic ratio between 1:1 and 4:1.

8. The neuroprotective composition for use according to the preceding claims wherein the composition is a supplement to a ketogenic diet.

9. Neuroprotective composition for use according to the preceding claims wherein the composition is a supplement to a ketogenic tube feed.

10. Neuroprotective composition for use according to claims 7 to 9 wherein the subject suffering from or at risk of seizures is administered the neuroprotective composition and provided with between 0.1 mg and 18 mg citrate gram of citrate per kcal of ketogenic diet and between 15 mg and 25 mg gram of pyruvate per kcal of ketogenic diet.

11. Neuroprotective composition for use according to claim 10 further providing nicotinamide riboside in an amount in a range of 0.01 mg to 0.4 mg per kcal of ketogenic diet, and/or leucine in an amount of 0.02 mg to 30 mg per kcal of ketogenic diet.

12. Neuroprotective composition for use according to the preceding claims wherein
citrate is present in the neuroprotective composition in an amount to provide a daily dosage of 0.01 gram to 0.7 gram per kilogram bodyweight per day;
pyruvate is present in the neuroprotective composition in an amount to provide a daily dosage of 0.15 gram to 1 gram per kilogram bodyweight per day; and
optionally nicotinamide riboside is present in the neuroprotective composition in an amount to provide a daily dosage of 0.5 mg to 15 mg per kilogram bodyweight per day;
and optionally leucine is present in the neuroprotective composition in an amount to provide a daily dosage of 3 mg to 30 mg per kilogram bodyweight per day.

## Patentansprüche

1. Neuroprotektive Zusammensetzung, die therapeutisch wirksame Mengen einer Kombination aus Pyruvat und Citrat umfasst, zur Anwendung bei der Behandlung und/oder Prävention von neuronalen Schäden bei einem Subjekt, das an Anfällen leidet oder dafür anfällig ist.

2. Neuroprotektive Zusammensetzung zur Anwendung gemäß Anspruch 1, wobei die Behandlung und/oder Prävention von neuronalen Schäden die Prävention und/oder Behandlung von Neuroinflammation, die Prävention und/oder Verzögerung der Bildung epileptiformer Schaltkreise im Gehirn einschließt.

3. Neuroprotektive Zusammensetzung zur Anwendung gemäß den Ansprüchen 1 und 2, wobei die Behandlung und/oder Prävention von neuronalen Schäden eine Erhöhung der Stoffwechselaktivität, vorzugsweise der Glykolyse und der mitochondrialen Sauerstoffverbrauchsrate von Neuronen und Gliazellen im Gehirn, umfasst.

4. Neuroprotektive Zusammensetzung zur Anwendung gemäß den voranstehenden Ansprüchen, wobei die Behandlung und/oder Prävention von neuronalen Schäden eine Verringerung der mit Anfällen verbundenen oder auf Anfälle folgenden Exzitotoxizität einschließt.

5. Neuroprotektive Zusammensetzung zur Anwendung gemäß den voranstehenden Ansprüchen, wobei das Subjekt, das an Anfällen leidet oder dafür anfällig ist, ein Subjekt ist, das an einem Schlaganfall, einer traumatischen Hirnverletzung, Migräne, Epilepsie, Alzheimer-Krankheit, Parkinson-Krankheit oder Erkrankungen leidet, die den Energie-Stoffwechsel von Neuronen und Gliazellen beeinträchtigen, einschließlich Glukosetransporter-Typ-1 (Glut1)-Mangel, Pyruvatdehydrogenase (PDH)-Mangel, Glykogenspeicherkrankheiten und mitochondrialen Erkrankungen.

6. Neuroprotektive Zusammensetzung zur Anwendung gemäß den voranstehenden Ansprüchen, wobei die Zusammensetzung weiterhin eine oder mehrere Verbindungen umfasst, die aus Leucin und Nicotinamid-Ribosid ausgewählt sind.

7. Neuroprotektive Zusammensetzung zur Anwendung gemäß den voranstehenden Ansprüchen, wobei das Subjekt, das an Anfällen leidet oder dafür anfällig ist, eine ketogene Diät einhält, vorzugsweise eine ketogene Diät mit einem ketogenen Verhältnis zwischen 1:1 und 4:1.

8. Neuroprotektive Zusammensetzung zur Anwendung gemäß den voranstehenden Ansprüchen, wobei die Zusammensetzung eine Ergänzung zu einer ketogenen Diät ist.

9. Neuroprotektive Zusammensetzung zur Anwendung gemäß den voranstehenden Ansprüchen, wobei die Zusammensetzung eine Ergänzung zu einer ketogenen Sondennahrung ist.

10. Neuroprotektive Zusammensetzung zur Anwendung gemäß den Ansprüchen 7 bis 9, wobei dem Subjekt, das an Anfällen leidet oder dafür anfällig ist, die neuroprotektive Zusammensetzung verabreicht wird und ihm zwischen 0,1 mg und 18 mg Citrat Gramm Citrat pro kcal ketogener Diät und zwischen 15 mg und 25 mg Gramm Pyruvat pro kcal ketogener Diät bereitgestellt werden.

11. Neuroprotektive Zusammensetzung zur Anwendung gemäß Anspruch 10, die zusätzlich Nicotinamid-Ribosid in einer Menge im Bereich von 0,01 mg bis 0,4 mg pro kcal ketogener Diät und/oder Leucin in einer Menge von 0,02 mg bis 30 mg pro kcal ketogener Diät bereitstellt.

12. Neuroprotektive Zusammensetzung zur Anwendung gemäß den voranstehenden Ansprüchen, wobei
Citrat in der neuroprotektiven Zusammensetzung in einer Menge vorhanden ist, um eine Tagesdosis von 0,01 Gramm bis 0,7 Gramm pro Kilogramm Körpergewicht pro Tag bereitzustellen;
Pyruvat in der neuroprotektiven Zusammensetzung in einer Menge vorhanden ist, um eine Tagesdosis von 0,15 Gramm bis 1 Gramm pro Kilogramm Körpergewicht pro Tag bereitzustellen; und
optional Nicotinamid-Ribosid in der neuroprotektiven Zusammensetzung in einer Menge vorhanden ist, um eine Tagesdosis von 0,5 mg bis 15 mg pro Kilogramm Körpergewicht pro Tag bereitzustellen;
und optional Leucin in der neuroprotektiven Zusammensetzung in einer Menge vorhanden ist, um eine Tagesdosis von 3 mg bis 30 mg pro Kilogramm Körpergewicht pro Tag bereitzustellen.

## Revendications

1. Composition neuroprotectrice comprenant des quantités thérapeutiquement efficaces d'une combinaison de pyruvate et de citrate, pour une utilisation dans le traitement et/ou la prévention de lésions neuronales chez un sujet souffrant ou présentant un risque de crises.

2. Composition neuroprotectrice pour une utilisation selon la revendication 1, où le traitement et/ou la prévention de lésions neuronales inclut la prévention et/ou le traitement de la neuroinflammation, la prévention et/ou le retardement de la formation de circuits épileptiformes dans le cerveau.

3. Composition neuroprotectrice pour une utilisation selon les revendications 1 et 2, où le traitement et/ou la prévention de lésions neuronales implique une augmentation de l'activité métabolique, de préférence de la glycolyse et du taux de consommation d'oxygène mitochondrial des neurones et des cellules gliales dans le cerveau.

4. Composition neuroprotectrice pour une utilisation selon les revendications précédentes, où le traitement et/ou la prévention de lésions neuronales inclut une réduction de l'excitotoxicité associée ou consécutive à des crises.

5. Composition neuroprotectrice pour une utilisation selon les revendications précédentes, où le sujet souffrant ou présentant un risque de crises est un sujet souffrant d'un accident vasculaire cérébral, d'un traumatisme crânien, de migraines, d'épilepsie, de la maladie d'Alzheimer, de la maladie de Parkinson ou d'affections altérant le métabolisme énergétique neuronal et glial, incluant un déficit en transporteur de glucose de type 1 (Glut1), un déficit en pyruvate déshydrogénase (PDH), des maladies de stockage du glycogène et des maladies mitochondriales.

6. Composition neuroprotectrice pour une utilisation selon les revendications précédentes, où la composition comprend en outre un ou plusieurs composés choisis parmi la leucine et le nicotinamide riboside.

7. Composition neuroprotectrice pour une utilisation selon les revendications précédentes, où le sujet souffrant ou présentant un risque de crises suit un régime cétogène, de préférence un régime cétogène avec un rapport cétogène compris entre 1:1 et 4:1.

8. Composition neuroprotectrice pour une utilisation selon les revendications précédentes, où la composition est un complément à un régime cétogène.

9. Composition neuroprotectrice pour une utilisation selon les revendications précédentes, où la composition est un complément à une alimentation cétogène par sonde.

10. Composition neuroprotectrice pour une utilisation selon les revendications 7 à 9, où le sujet souffrant ou présentant un risque de crises est administré la composition neuroprotectrice et reçoit entre 0,1 mg et 18 mg de citrate gramme de citrate par kcal de régime cétogène et entre 15 mg et 25 mg gramme de pyruvate par kcal de régime cétogène.

11. Composition neuroprotectrice pour une utilisation selon la revendication 10, fournissant en outre du nicotinamide riboside en une quantité comprise dans une plage de 0,01 mg à 0,4 mg par kcal de régime cétogène, et/ou de la leucine en une quantité de 0,02 mg à 30 mg par kcal de régime cétogène.

12. Composition neuroprotectrice pour une utilisation selon les revendications précédentes, où
le citrate est présent dans la composition neuroprotectrice en une quantité permettant de fournir une dose quotidienne de 0,01 gramme à 0,7 gramme par kilogramme de poids corporel par jour ;
le pyruvate est présent dans la composition neuroprotectrice en une quantité permettant de fournir une dose quotidienne de 0,15 gramme à 1 gramme par kilogramme de poids corporel par jour ; et
éventuellement, le nicotinamide riboside est présent dans la composition neuroprotectrice en une quantité permettant de fournir une dose quotidienne de 0,5 mg à 15 mg par kilogramme de poids corporel par jour ;
et éventuellement, la leucine est présente dans la composition neuroprotectrice en une quantité permettant de fournir une dose quotidienne de 3 mg à 30 mg par kilogramme de poids corporel par jour.
